# EUROPEAN PATENT SPECIFICATION

(11) **EP 2 852 403 B1**
(45) Date of publication and mention of the grant of the patent: **07.03.2018**
(21) Application number: 13784517.8
(22) Date of filing: 26.04.2013
(51) Int. Cl.: A61K 38/48

(54) **METHODS FOR MODIFICATION OF TISSUES**
VERFAHREN ZUR MODIFIZIERUNG VON GEWEBE
PROCÉDÉS POUR UNE MODIFICATION DE TISSUS

(30) Priority: 30.04.2012 US 201261640008 P
(43) Date of publication of application: 01.04.2015
(73) Proprietor: Elastomics AB, S-144 62 Rönninge (SE)
(72) Inventor: ROZKOV, Aleksei, S-144 62 Rönninge (SE)
(74) Representative: Brann AB
(86) International application number: PCT/SE2013/050472
(87) International publication number: WO 2013/165304

(56) References cited:
- WO-A1-90/12590
- WO-A1-95/31902
- WO-A1-99/30730
- WO-A1-2010/102262
- WO-A2-2004/006864
- WO-A2-2004/073504
- WO-A2-2009/111083
- DE-A1- 3 719 367
- GB-A- 2 323 530
- US-B1- 6 194 189
- YUAN-PING HAN: "Matrix metalloproteinases, the pros and cons, in liver fibrosis", JOURNAL OF GASTROENTEROLOGY AND HEPATOLOGY, vol. 21, no. s3, 1 October 2006 (2006-10-01), pages S88-S91, XP055228632, AU ISSN: 0815-9319, DOI: 10.1111/j.1440-1746.2006.04586.x
- DIETMAR ULRICH ET AL: "Matrix Metalloproteinases and Tissue Inhibitors of Metalloproteinases in Sera and Tissue of Patients with Dupuytren's Disease", PLASTIC AND RECONSTRUCTIVE SURGERY., vol. 112, no. 5, 1 October 2003 (2003-10-01), pages 1279-1286, XP055228642, US ISSN: 0032-1052, DOI: 10.1097/01.PRS.0000081462.40448.49
- P. LU ET AL: "Extracellular Matrix Degradation and Remodeling in Development and Disease", COLD SPRING HARBOR PERSPECTIVES IN BIOLOGY, vol. 3, no. 12, 14 September 2011 (2011-09-14), pages a005058-a005058, XP055228703, DOI: 10.1101/cshperspect.a005058
- L. M. COUSSENS ET AL: "Inflammatory mast cells up-regulate angiogenesis during squamous epithelial carcinogenesis", GENES AND DEVELOPMENT., vol. 13, no. 11, 1 June 1999 (1999-06-01), pages 1382-1397, XP055228940, US ISSN: 0890-9369, DOI: 10.1101/gad.13.11.1382
- RODERFELD M ET AL.: 'Inhibition of hepatic fibrogenesis by matrix metalloproteinase-9 mutans in mice' FASEB JOURNAL vol. 20, 2006, pages 444 - 454, XP055120566

## Description

### Field of the invention

The present invention relates to the field of enzymatic modification of tissues, and especially to the use of eukaryotic endopeptidases for modification of mechanical properties or the shape of mammalian tissue.

### Background

Mechanical properties and shape of mammalian tissues are defined by extracellular matrix.

Mechanical properties of tissues tend to change with age or due to disease. Increased organ stiffness and fragility is cause to multiple pathological conditions, such as: liver fibrosis (Wells 2005; Georges, Hui et al. 2007), liver cirrhosis (Dechene, Sowa et al. 2010), adhesive capsulitis , Dupuytren's contracture(Hurst, Badalamente et al. 2009), phimosis, aged eye lens stiffness (Hansen, Stitzel et al. 2003; Stitzel, Hansen et al. 2005).

Stiffness of healthy organs sets limitations on (1) ability to reshape tissues, desired for plastic and reconstructive surgery, (2) ability of transplant tissue to adjust to shape of host tissue without gaps, (3) ability to increase amount of transplanted tissue by means of stretching, (4) ability for scarless healing of lesions and wounds, (5) ability to heal ruptured tissues.

Stiffness of organs sets limitations on ability of organs to expand (grow). Notably, cell cultures, comprising the tissues, have high capacity for proliferation in vitro and in vivo, however, whole tissues and organ have far more limited ability to expand due to extracellular matrix stiffness.

Stiffness of extracellular matrix is known to define cell behavior, such as:
adhesion, migration, proliferation and even differentiation patterns.
Modification of tissue stiffness in situ could be the key to regulate cell behavior, including differentiation, within natural environment of the cell (Discher, Janmey et al. 2005; Engler, Sen et al. 2006; Engler, Sweeney et al. 2007).

Proteolytical enzymes have been used for decades in medicine, biotechnology and industry in order to degrade extracellular matrix and thus affect properties of tissues. Collagenolytic enzymes of bacterial source (Clostridium histolyticum) and crab digestive collagenases are used in medicine for decades. Those collagenases are highly efficient for tissues dissociation, effects being used for necrotic tissues debridement (Klasen 2000), scar removal, derivation of primary cell suspensions from tissues, islet derivation from pancreases (Li, Yuan et al. 2009).

However, use of bacterial collagenolytic enzymes envolves a risk of ruptures for treated and adjacent tissues. Collagenase from Clostridium histolyticum, when applied to Dupuytren's cords in vitro allowed 93% decrease in tensile modulus and 3,600 units dosage; however, only 300 units dose of collagenase was sufficient for cords rupture within physiological cords limits (Starkweather, Lattuga et al. 1996). Clinical trials of collagenase from Clostridium histolyticum demonstrated increase of limb mobility range after collagenase treatment, however, accompanied by adverse effects caused by integrity failure in adjacent tissues (such as: tendon rupture, blistering, pain, numbness, etc) (Hurst, Badalamente et al. 2009).

There are a number patents and scientific articles covering the use of bacterial collagenase or crab/shrimp digestive collagenase and proteolytic enzymes in surgery, regenerative medicine, cosmetology, food industry, etc.

The majority of work on MMP's and other tissue-remodelling enzymes is related to their role in cancer and mostly cover their molecular structure, search for inhibititors or use as diagnostic markers.

However, the use of MMP's and other tissue-remodelling enzymes for use in the applications for which bacterial and digestive collagenases are known has not received much attention.

There are multiple patents where a certain effect, achieved with bacterial collagenase was speculatively extended into all types of collagenolytic enzymes. This includes US patent 6,365,405, US patent 8,323,642, US 20080145357. The experimental work disclosed in these documents do however not include mammalian tissue-remodeling enzymes.

Work by Hirayasu (Hirayasu, Yoshikawa et al. 2008) shows mammalian extracellular matrix -degrading enzyme Granzyme B use to detach cells in vitro.

Use of enzymes to degrade fibrin clot, scar, fat deposits, which are temporary tissues, is also known in the art.

US patent 5,922,322 and US patent application 20110091436 disclose that fibrin clot can be dissolved by MMP-3 and by MMP-10, respectively. Fibrin is a short-term temporary tissue, it is meant by nature to be dissolved after short while. Use of MMP's allows to increase the rate of dissolving the fibrin clot, therefore, disruption and desintegration effects are highly desirable.

EP0637450 claims that protelytical enzymes, including those of MMP family, can be used "to provide partial degradation of either scar tissue or matrix component", being part of sofnening, expanding composition. Human stromelysin and collagenase, purified from human gingival fibroblasts, together with other components of softening composition, were injected into scar in order to make it more loose and soft. Scar is a temporary tissue intended to interconnect gaps between ruptured permanent tissue, however, the rate of natural dissolving of scars is too low and enzymes are required to enhance the process.

US patent application 20100247512 suggests that enhancing MT1-MMP secretion by host organism (patient's) own cells may enable fat deposition and inhibiting of MT1-MMP inhibits process of fat deposition.

WO 2009111083 describes that cathepsin L, when injected subcutaneously, partially dissolves extracellular matrix of subcutaneous fat deposits (fibrous septae).

US patent 7,935,337 teaches that MMPs (namely, MMP-3 and MMP-7), when applied to herniated intervertebrate cartilage discs can partially degrade it, both in vivo and in vitro. Authors discovered that (1) "using MMP-3, MMP-7 or both in combination significantly reduced the wet weight compared to the control" and (2) "resorption of herniated disc" that was treated by those MMPs was evaluated by histochemistry staining of MMP-treated tissues with Safranin O. Authors evaluate success of the method in terms of tissue dissociation (resorbtion, degradation) efficiency. The purpose of the method described by authors is reverse to effect of tissue maintenance and functional integrity that is the subject of the present invention.

US patent application 20110044960 describes MMP-3 being delivered into tooth. Authors demonstrate proliferative, migrative, anti-apoptotic effect of MMP-3 on vascular endothelial cells in vitro and induction of odontoblasts by MMP-3 in vitro. Authors observed same benefectory effects when they applied MMP-3 to tooth in vivo, thus demonstrating stimulatory effect of MMP-3 on vascular endothelial and odonthoblast cells. However, authors did not demonstrate effects of changing mechanical or shape properties of actually tooth tissue (such as to correct positioning of tooth or to increase it's plasticity).

Prior art for using Tissue-Remodeling Enzymes on mammalian tissues are reviewed in **Table 4.**

### Summary

The present invention relates to the use of pre-activated human MMP-9 to provide a modification of the mechanical properties of mammalian connective tissue while the tissue remains cohesive, wherein the MMP-9 has been pre-activated by 4-aminophenylmercuric acetate (APMA) activation and wherein the MMP-9 is incubated *ex vivo* with samples of mammalian connective tissue.

In one aspect, the invention relates to a method for modification of mechanical properties of a mammalian tissue, excluding scar, blood clot, fat; comprising a step of contacting said tissue with an isolated eukaryotic endopeptidase in an amount sufficient to cause a modification of mechanical properties of said tissue, but insufficient to cause degradation of said tissue's into incohesive parts.

Currently preferred embodiments are set out in the dependent claims.

### Figures

Figure 1: Functional integrity of muscle and tendon. Loss of functional integrity may be result in pathologies, such as: pain, numbness, paralysis, muscle rupture, congenital muscular dystrophy or tendon rupture.
Figure 2: Functional integrity of skin. Loss of functional integrity may result in pathologies, such as: wound, lesion, rupture, ulcer, epidermolysis bullosa, bullous pemphigoid, blistering, alopecia.
Figure 3. Plastic materials are less prone to rupture and erosion, compared to stiff ones materials. Mechanisms of plasticity, brittleness (fragility), rupture and erosion are reviewed and illustrated in (Picu R.C. (2011), Hong W. et.al. (2008), Wang X. et.al. (2012)).

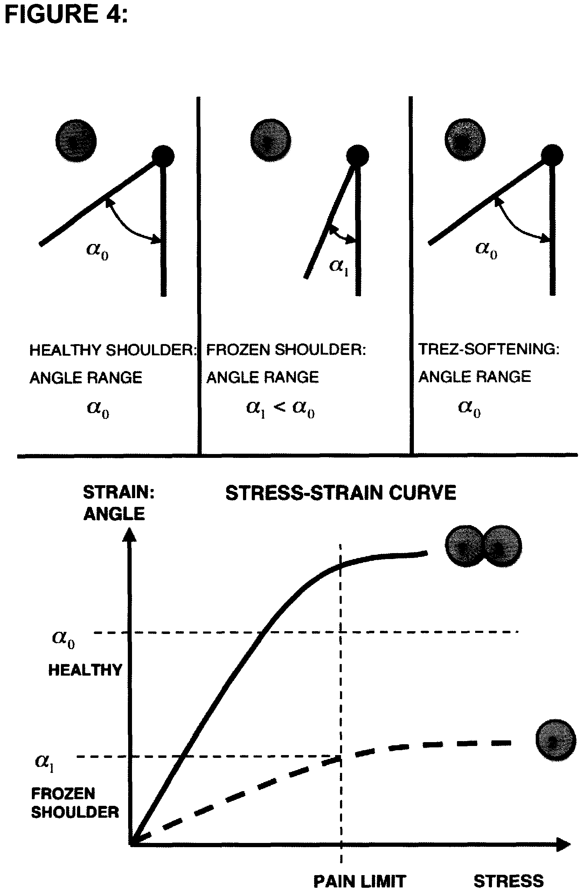

Figure 4: Adhesive capsulitis: stress-strain characteristics. Reduced stress-strain ratio (as after TREZ-treatment) allow to increase motility range. Implications for adhesive capsulitis (frozen shoulder).

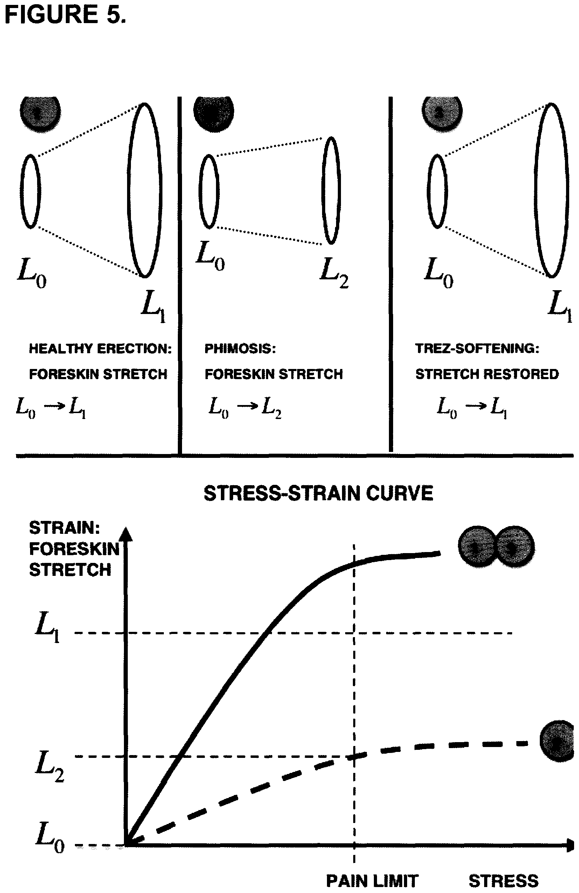

Figure 5: Phimosis: stress-strain characteristics. Reduced stress-strain ratio (as after TREZ-treatment) allow to increase painless deformation range. Implications for phimosis.

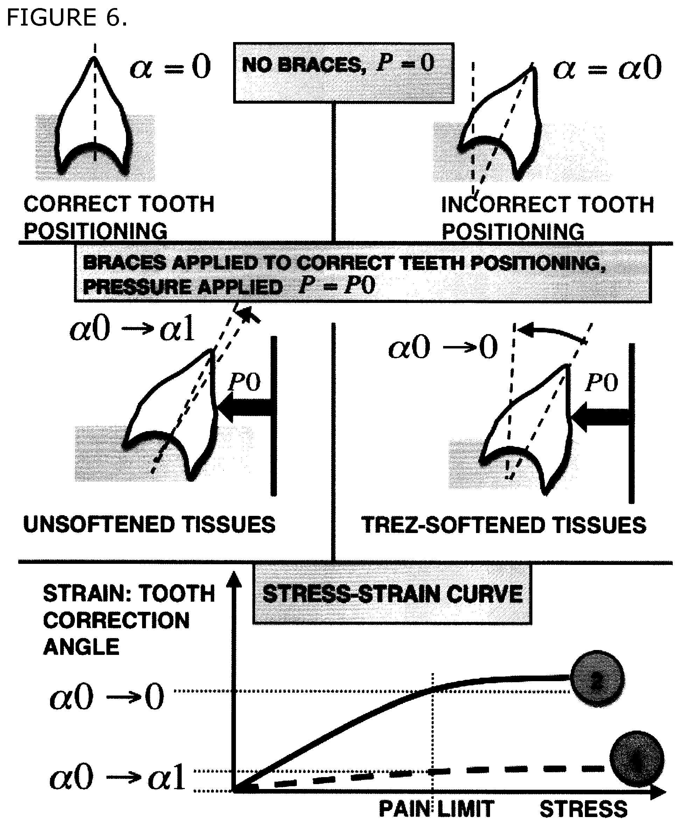

Figure 6: Dental braces method: stress-strain characteristics. Reduced stress-strain ratio (as after TREZ-treatment) allow to increase painless deformation range. Implications for teeth positioning correction using dental braces (continued: figure 9B).
Figure 7: Cohesive tendon rearrangement, scheme. Cohesive rearrangement of tendon by MMP-9, compared to non-cohesive split by aggressive collagenases. Cohesive split allows to improve permeability.
Figure 8. MMP-9 allows cohesive tendon rearrangement (I) (microphotographs). Large limbal tendon, treated by MMP-9, 1 mg/ml improves plasticity and permeability and reduces stiffness, however, maintaining functional integrity.
   Top panel: limbal tendon, treated by MMP-9, before and after treatment. The treated tendon is split into multiple interconnected fibrils thus reducing tendon stiffness, enhancing plasticity and permeability. Importantly, the individual fibrils remain interconnected as meshwork, therefore, the tendon remains a one whole entity.
   Middle panel: limbal tendon, treated by buffer, retains stiffness, inability to plastic modification and of low permeability due to dense packing.
   Bottom panel: limbal tendon, treated by collagenase from Clostridium histolyticum, 1 mg/ml, before and after treatment. The treated tendon is split into multiple floating microscopic particles, therefore, functional integrity is lost.

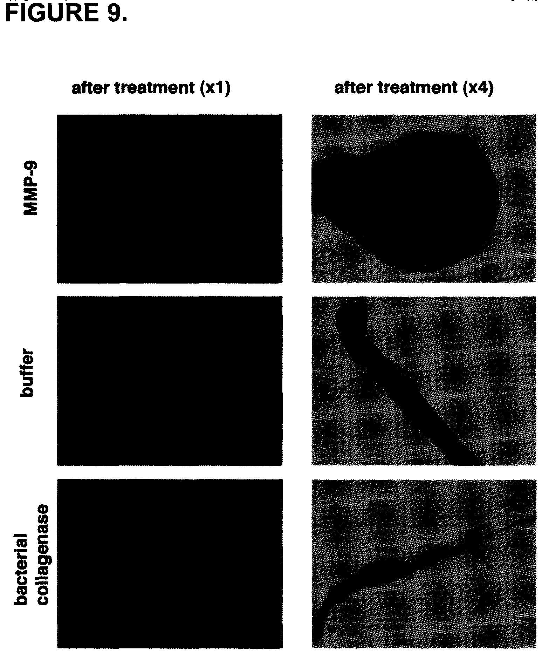

Figure 9. MMP-9 allows cohesive tendon rearrangement (II) (microphotographs). Large limbal tendon, treated by MMP-9 and by collagenase from Clostridium histolyticum: differences in plastic deformation vs integrity.
   Top panel: tendon, treated by MMP-9, 0.1 mg/ml, is split into multiple interconnected fibrils thus reducing tendon stiffness, enhancing plasticity and permeability. Importantly, the individual fibrils remain interconnected as meshwork, therefore, the tendon remains a one whole entity.
   Middle panel: limbal tendon, treated by buffer, retains stiffness, inability to plastic modification and of low permeability due to dense packing.
   Bottom panel: limbal tendon, treated by collagenase from Clostridium histolyticum, 0,1 mg/ml, before and after treatment. The treated tendon is partially degraded into multiple floating microscopic particles. The remaining, undigested part of tendon, remains thin and prone to rupture; however, plasticity is not improved (remaining fibrils within the tendon are still tightly packed).

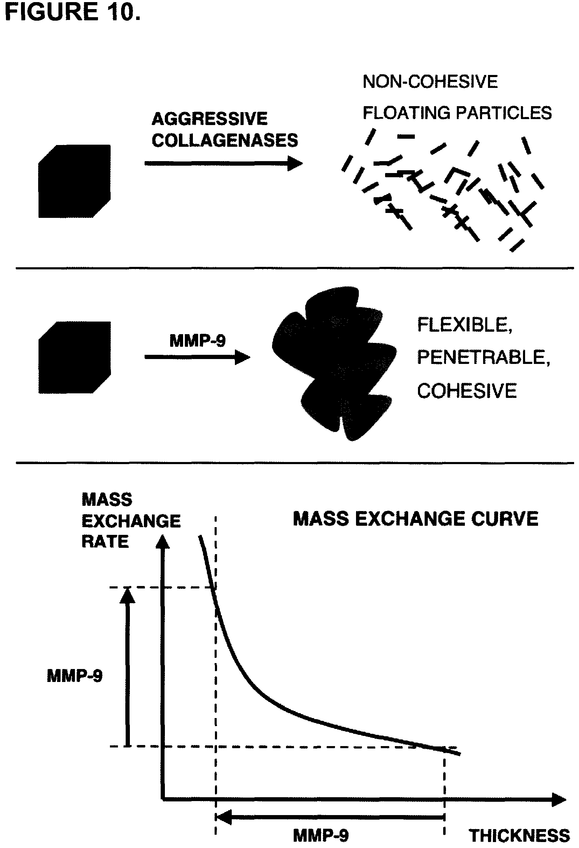

Figure 10: Cohesive pancreas rearrangement, scheme. Cohesive split of pancreas by MMP-9, compared to non-cohesive split by aggressive collagenases. Cohesive split allows to improve permeability.

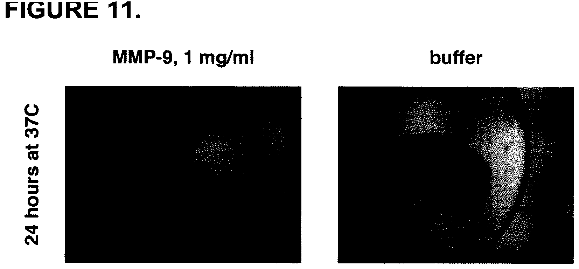

Figure 11. MMP-9 allows cohesive pancreatic tissue rearrangement (microphotographs). MMP-9 treatment splits pancreas tissue from solid piece into thin interconnected lobules with intact duct system.
   Left panel: pancreas tissue specimen, treated by MMP-9.
   Right panel: pancreas specimen of same size, treated by buffer.
      Both samples were treated for 24 hours at 37C and were examined under phase contrast microscope. MMP-9 treatment splits pancreatic tissue into thin interconnected lobules (whole system of exocrine ducts, lumens and local cell niches remains intactact and tissue remains one whole entity). Reduction of tissue specimen thickness improves permeability of vitro cultures.

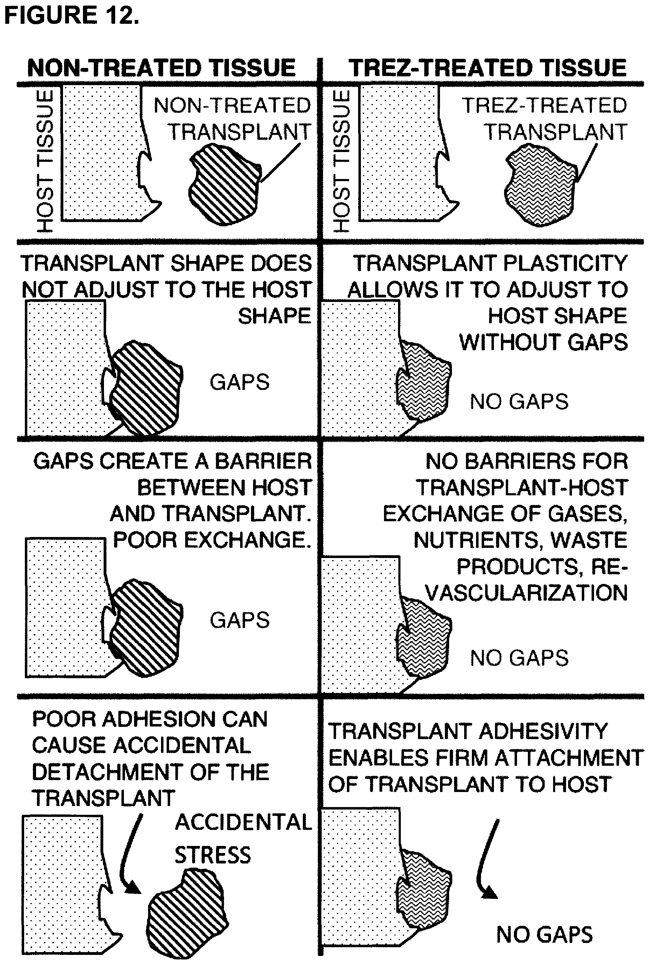

Figure 12: Plasticity and adhesivity: effect in transplantation and implantation. Improved plasticity and adhesivity of TREZ-treated transplanted tissues allows to improve process of implantation.

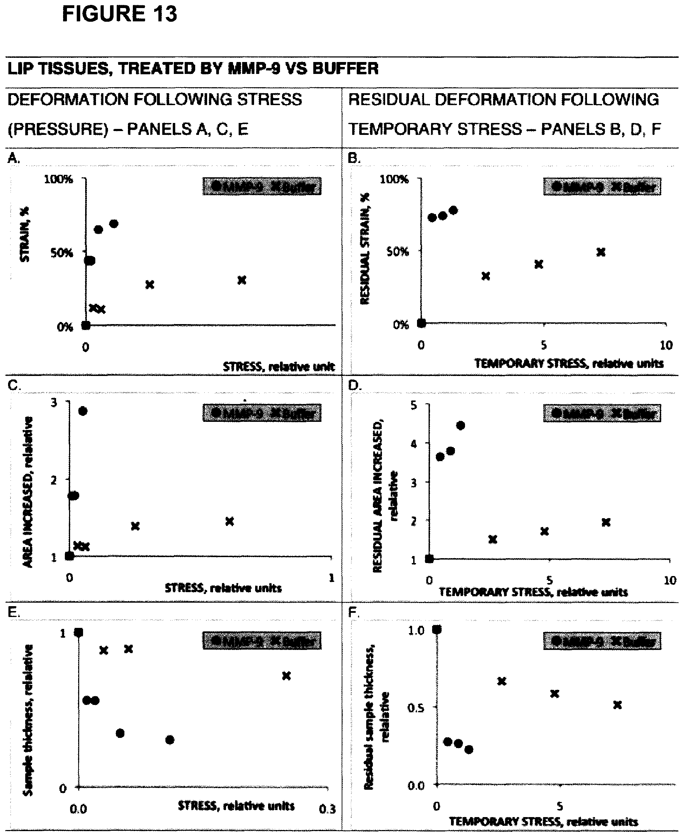

Figure 13: Lip tissue, treated by MMP-9 vs buffer (quantitive stress-strain characteristics).
   Panels A, B (top): Stress-strain curve (X: stress applied to the tissue sample, in relative units; Y: resulting strain of the tissue sample, in %)
   Panels C, D (middle): Relative area of tissue sample resulting from stress applied (X: stress, in relative units; Y: relative area of the tissue sample)
   Panels E, F (bottom). Thickness of the tissue sample resulting from stress applied (X: stress, in relative units, Y: relative thickness of the tissue sample)
   Left panels demonstrate stress-strain characteristics of tissue, resulting from permanent stress applied. Right panels describe plasticity: residual strain and deformation characteristics after temporary stress (pressure) applied and removed.

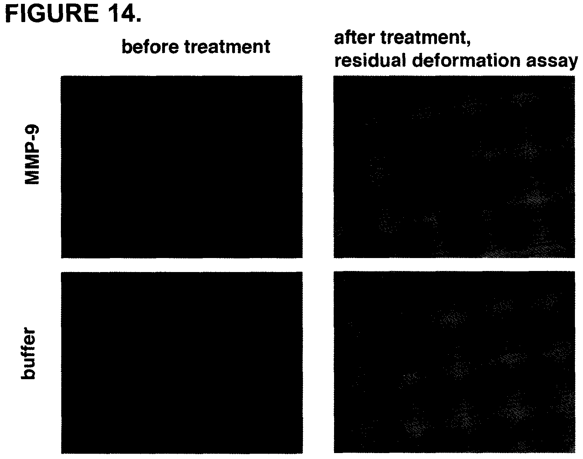

Figure 14. Increased plasticity of lip connective tissue after MMP-9 treatment (microphotographs). MMP-9 treatment of subcutaneous connective tissues of lip renders them highly plastic. Top panel: MMP-9, 0.1 mg/ml, treated tissues of lip. Bottom panel: buffer-treated tissues of lip. Both specimen were treated for 4 days at 37C and subject by temporary stress assay.

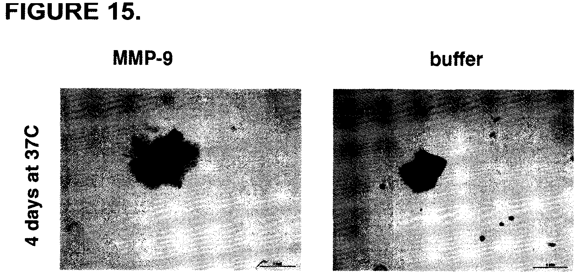

Figure 15. MMP-9 treatment of subcutaneous connective tissues of lip renders them gel-like and adhesive.
   Top panel: MMP-9, 0.1 mg/ml, treated tissues of lip. Bottom panel: buffer-treated tissues of lip. Both specimen were treated for 4 days at 37C and were subject to qualitative tests. MMP-9 treated sample is more loose, easily adjusts it's shape, is highly adhesive and behaves like a viscous, rather than elastic, body in creep assay.

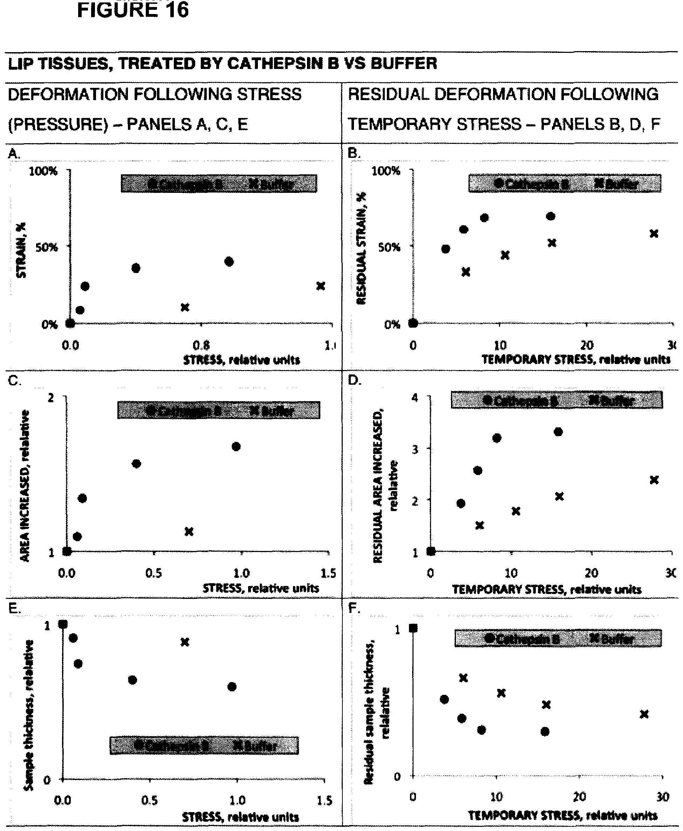

Figure 16: Lip tissues, treated by Cathepsin B vs buffer (quantitive stress-strain characteristics). See figure 13 for detailed description.

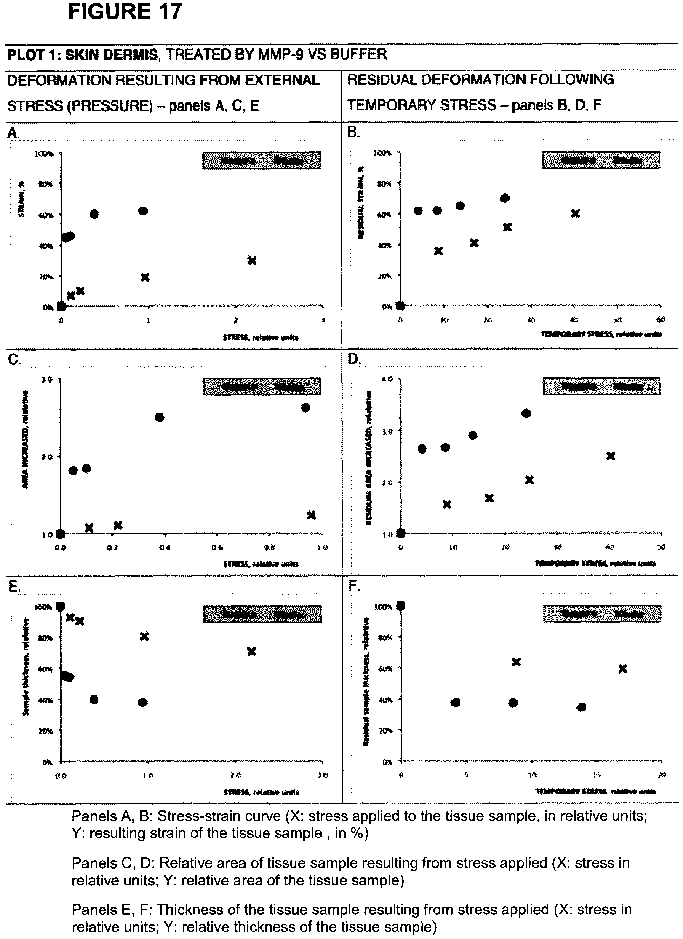

Figure 17: Dermis, treated by MMP-9 (quantitive stress-strain characteristics). See figure 13 for detailed description.

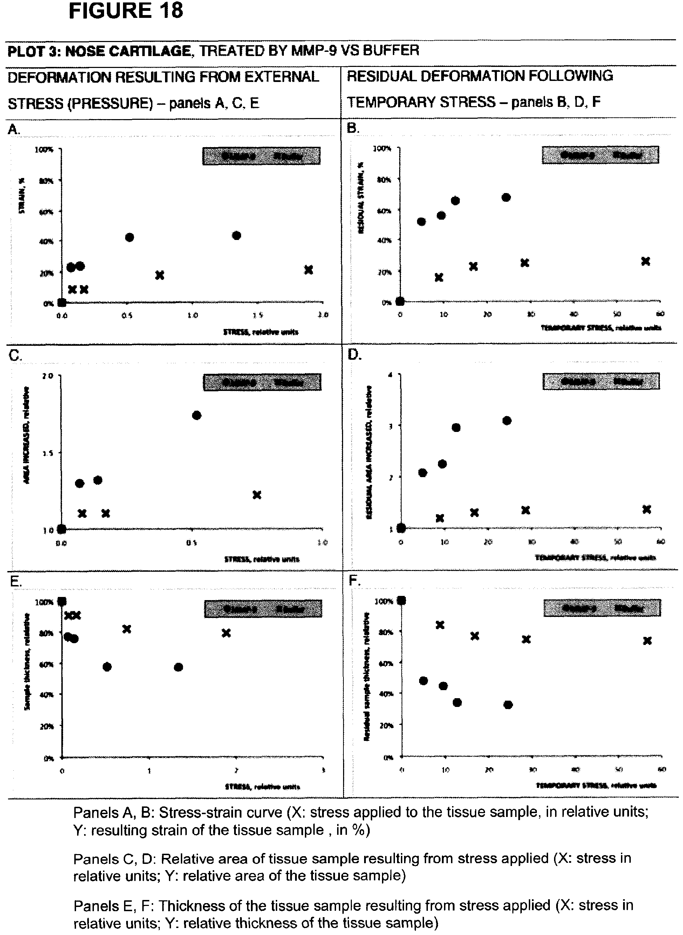

Figure 18: Cartilage, , treated by MMP-9 (quantitive stress-strain characteristics). See figure 13 for detailed description.

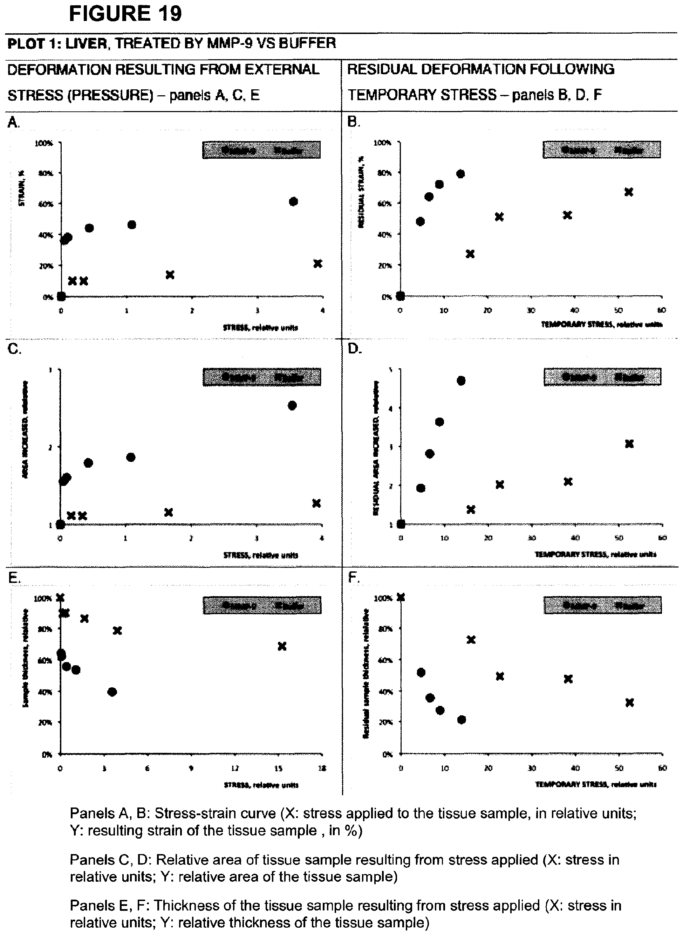

Figure 19: Liver, treated by MMP-9 (quantitive stress-strain characteristics). See figure 13 for detailed description.

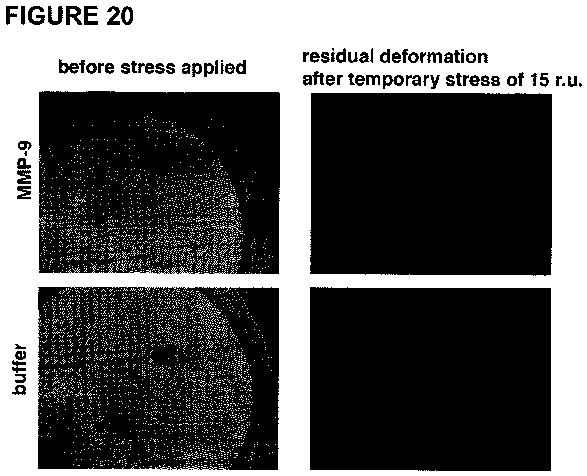

Figure 20. MMP-9 treatment of liver tissue in vitro makes it (1) less stiff, (2) more plastic and (3) more cohesive, compared by untreated liver tissue.
   MMP-9 (top) and buffer-treated (bottom) tissue samples before any stress (load) applied (left panels) vs after temporary stress of 15 relative units is applied. Noticably, MMP-9 -treated liver tissue is more cohesive and less prone to ruptures under stress.

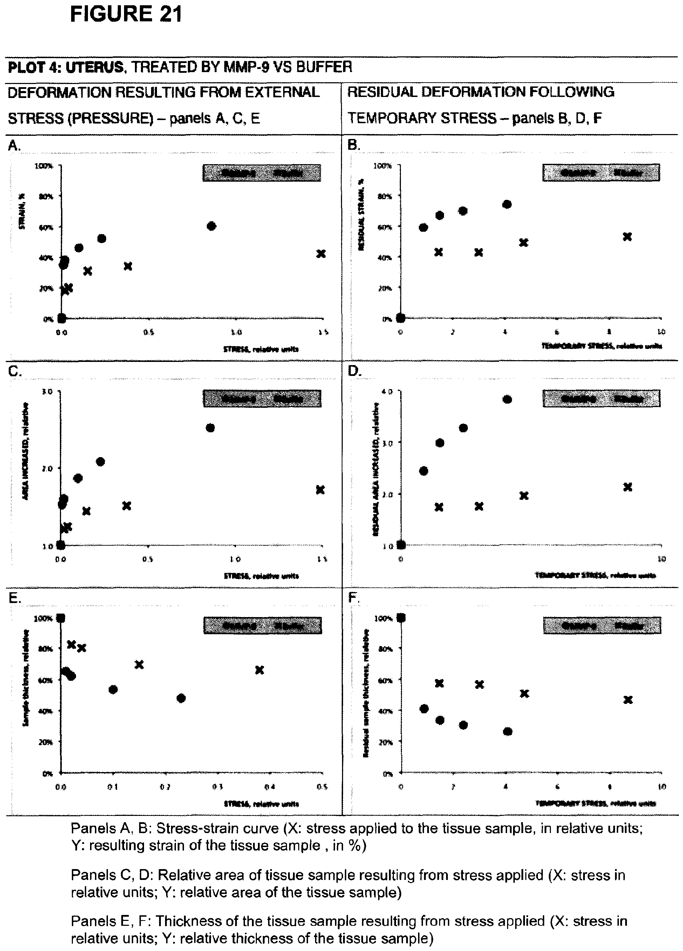

Figure 21: Uterus tissues, treated by MMP-9 (quantitive stress-strain characteristics). See figure 13 for detailed description.

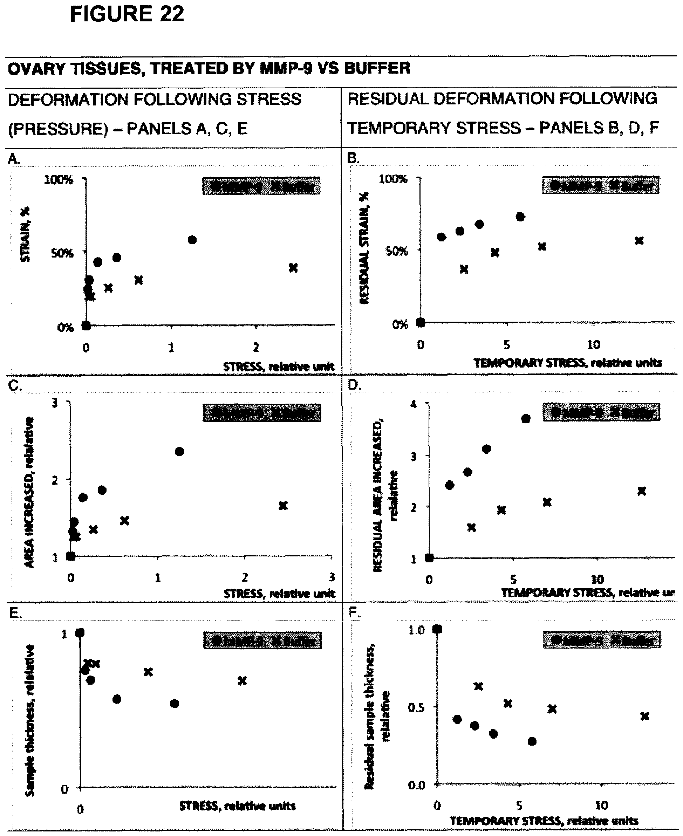

Figure 22: Ovary, treated by MMP-9 (quantitive stress-strain characteristics). See figure 13 for detailed description.

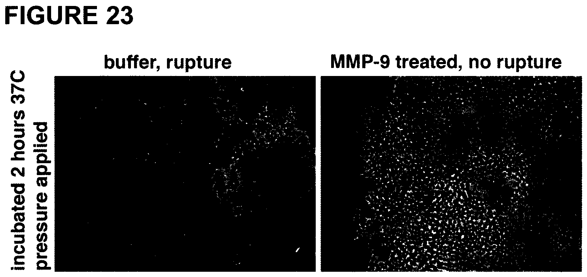

Figure 23. Islet: fragility assay. MMP-9 treated pancreatic insulin-producing islets of Langerhans are (1) better spread under load, and (2) less prone to rupture.
   Left panel: buffer treated islet: rupture under load. Right panel: MMP-9 treated islet is spread under load from spherical formation into thin layer, the cells within the islet remain cohesive, the isled does not undergo rupture under load.

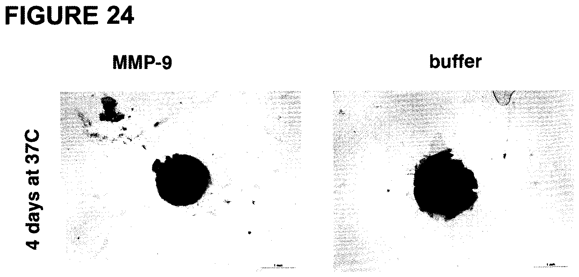

Figure 24: Eye lens, fragility assay. MMP-9 renders eye lens more resistant to rupture under stress applied.
   Left panel: eye lens, treated by MMP-9, 0.1 mg/ml. Right panel: eye lens of same animal, treated by buffer. Both samples were treated for 4 days at 37C and were subject to temporary pressure of same intensity and duration. MMP-9 treated sample reacted to pressure by elastic deformation, while buffer-treated sample reacted in ruptures.

### Detailed description of the invention

Although current methods using proteolytic enzymes in medicine are highly efficient in applications that aim to dissociate or otherwise destruct tissues (such as scars, necrotic tissues of burn wounds, contracted cords), there is an unmet need related to modification of mechanical or geometrical properties of tissues while maintaining functional integrity of treated and adjacent tissues.

According to the invention, pre-activated human MMP-9 is used *ex vivo* to modify mechanical properties of mammalian connective tissues while cohesion of treated tissues remain on level sufficient to preserve tissue function.

The present description demonstrates effects of: reduced stiffness, reduced stress-strain ratio, increased plasticity, increased adhesiveness, reduced fragility on a wide range of mammalian tissues, after treatment by TREZ and having direct implementation for transplantation, plastic and reconstructive surgery.

We have discovered that pre-activated human MMP-9 has the ability to modify mechanical properties and shape of permanent tissues while maintaining their functional integrity.

The following changes in TREZ-treated tissues were observed:
(I) tissues became more elastic: (1) reduced stress-strain ratio (reduced stiffness), (2) increased plasticity (ability to adapt shape; residual deformation after temporary stress), (3) reduced fragility (brittleness). Less pressure was required to achieve certain deformation after TREZ-treatment. Fixed pressure resulted in higher extent of tissue deformation after TREZ-treatment.
(II) tissues became more plastic (gel-like): (1) tissues, that used to behave like elastic solid materials prior to treatment, after TREZ treatment behaved like gels, (2) increased viscoelasticity, (3) reduced risk for ruptures, (4) reduced risk for erosion, (5) increased adhesivity to surfaces and other tissues, (6) increased ability to modify shape, (7) increased cohesion within treated tissue.
(III) modification of shape: (1) increased ability to stretch (increase area), (2) increased ability to reduce thickness, (3) increased ability to change shape, (4) increased ability to mold into required shape, (5) improved permeability within treated tissue sample.
(IV) integrity maintenance for adjacent tissues: (1) nerve integrity, (2) tendon integrity, (3) muscle integrity, etc.

The effects were observed for various tissue types of mammalian tissues: skin, tendon, cartilage, liver, pancreas, ovaries, pancreatic islets of Langerhans, muscle, eye lens, connective tissues of penis, uterus, lip and other tissues. All the tissues maintained functional integrity while treated by such compositions of tissue-remodeling enzymes that enabled modification of tissue stiffness, plasticity, adhesivity and other mechanic properties. It is important that enzyme formulation, sufficient for plastic deformation of one tissue, would not rupture or damage integrity of adjacent tissues, such as nerve.

Further we shall describe implementations of tissue-remodeling enzyme for increased plasticity and adhesivity of tissues in transplantation

### Extracellular matrix and collagen-based scaffold

### Definitions of mechanical properties

Here we provide definitions of mechanical properties used throughout this patent. Mechanical properties include the following and closely related (with synonyms) terms: elasticity, plasticity, fragility, viscoelasticity, adhesiveness, stiffness, integrity, stress-strain curve.

The mechanical properties are used to describe the state of the tissues and their response to stress.

The stress is a force, pressure or shear applied to the tissue. The response is measured as a strain or deformation of the tissue. The various stress loads and corresponding strain values can be plotted against each other and are referred here as "stress-strain curve".

A solid body is characterized as stiff if the strain-stress curve is steep, i.e. little deformation is tolerated. If the stress is higher than rupture barrier then the solid body breaks (loses cohesiveness or integrity) and body can be described as fragile.

A solid body is characterized as soft if the stress-strain curve is shallow, i.e. allows large strain (deformation).

A solid body is characterized as elastic when the deformation is reversible if the stress is removed. The stress-strain curve in case of elastic bodies have little or no hysteresis.

A solid body is characterized as plastic or gel-like when deformation is not reversible if the stress is removed. The stress-strain curve in case of plastic bodies have large hysteresis. Deformation of plastic (gel-like) bodies can also be tested in "creep test".

### Mechanical properties and shape of extracellular matrix

Extracellular matrix plays an important part in providing a stiffness, shape, integrity, shape and architectural organization of mammalian tissues and consists of several proteins (collagens, laminins, perlecans, agrins etc) and proteoglycans (Aumailley et.al. 1998). Stiffness of extracellular matrix sets limitations upon organs ability to expand (e.g. cirrhotic liver), to move within certain range (e.g. adhesive capsulitis), to attain a different shape (e.g. wrinkles). Stiffness of extracellular matrix is capable of defining differentiation patterns of cells (Engler 2004, Cell), to stimulate or restrict migration of certain cell types.

Rigidity and shape is mainly maintained by collagen scaffold, which is formed by thick and dense cross-linked fibrils (Aumailley et.al. 1998). Crosslinking of collagen fibrils increases with age with tissues becoming more fragile and less elastic, as often seen in skin, eye lens, bones etc.

There are several types of collagen, which properties may differ, with some of the collagen types being tissue-specific. Tendon is composed of different molecules assembled into different architecture compared to pancreatic islet or cartilage.

### Architectural organization

Extracellular matrix provides not only durability and elasticity, but also architecture and compartmentalization of a complex tissue. For tissues like liver, pancreatic insulin-producing beta islets, ovaries and testis it is essential for their function.

### Highly conserved between species

Extracellular matrix and collagens are conservative between species. That is why animal collagens are used in medicine. They have reduced risk of immune reaction and rejection.

### Highly durable: maintains stiffness and shape in living or dead tissues.

Extracellular matrix and collagen scaffolds are extremely durable. Turnover of collagen fibrils in organism is extremely slow (years) compared to turnover rate of non-extracellular matrix molecules. Collagen scaffolds maintain their architecture, durability, shape and integrity for years, in living and non-living tissues.
the terms "stress", "strain" and "stress-strain curve" can be interpreted in medical applications as follows:
Stress is a force applied to move the tissue. The strain is an actual tissue movement as a result of the applied stress. In treated tissues the stress-strain ratio is lower than in non-treated. It is either because less force is needed to move the tissue within the normal range, or because the range of movement is limited and forcing the tissue further is difficult and painful.

Treatment with TREZ would allow to soften the tissues and allow their faster remodelling and healing, while preserving a functional integrity

Stiffness of extracellular matrix is known to define cell behavior, such as: adhesion, migration, proliferation and even differentiation patterns. Modification of tissue stiffness in situ could be the key to regulate cell behavior, including differentiation, within natural environment of the cell (Discher, Janmey et al. 2005; Engler, Sen et al. 2006; Engler, Sweeney et al. 2007).

### Collagenolytic enzymes in medicine, biotechnology and industry.

Proteases are used in medicine, biotechnology, cosmetics, veterinary, food industry and other areas for decades (US patent 3003917 by Beiler et.al., dated 1961) . Examples include wound debridement (Klasen 2000), pancreatic islets derivation (Li, Yuan et al. 2009), cell culture (trypsin), proteolytical enzymes are used for meat softening, hide processing and other industries.

Collagenases are a subset of proteases that have collagen as as a preferred substrate. We classify collagenases into following three categories
1. Bacterial collagenases - mostly pathogenic (reviewed Harrington, 1996 Infection and Immunity 64-6). Example: Clostridium histolyticum (gangrene)
2. Digestive collagenases from crab and shrimp (for example: one derived from hepatopancreas of crab).
3. Tissue-remodeling collagenases of vertebrate/mammalian/human organisms produced by host organism in order to remodel host's own tissues for healthy purposes, such as: organ growth, regenerative organ remodeling, reproduction-wise tissues remodeling.

Although these types of collagenases act on same substrate, their effect on the tissues is very different, which is most easily understood from their intended purpose.

### Bacterial and digestive (aggressive) collagenases

Biological function of bacterial collagenases is to allow pathogenic bacteria to invade and spread into host tissue. Expression of bacterial collagenases allows pathogenic bacteria to pass through mammalian protection barriers in the body. As Clostridium bacteria (including Clostridium histolyticum) cause gas gangrene, the bacteria making collagenase don't care about maintaining health of the host organism. In our experiments we see that collagenase from Clostridium histolyticum digests mammalian tissues into microscopic, non-cohesive parts or to cell suspension.

Biological function of digestive collagenases (usually, of crab, shrimp or fish) is to digest any collagen-containing tissue into nutrients. Therefore, there is no biological reason for crab or shrimp to develop digestive collagenase that would preserve safe mammalian tissues and cells.

We suggest to unite all the collagenase that don't care for maintaining architecture and safe function of mammalian tissues under the name of aggressive collagenases. There is no evolutionary or physiological reason that those enzymes would preserve function, architecture and proper structure of mammalian tissues. On the contrary, aggressive enzymes are very efficient into digesting collagen scaffold into small, non-cohesive parts because that would fulfill the biological purpose of organism secreting those enzymes.

### Tissue-Remodeling Enzymes (TREZ)

However, nature created a number of proteolytical enzymes that can remodel mammalian (and other eukaryotic) tissues with high efficiency, however, being non-hazardous for the organism. Some of this enzymes are needed for organism growth (especially in embryonic and early postnatal age), some are needed for adult tissues remodeling (such as ovaries, uterus, mammary glands following onset of puberty, menstrual cycle, pregnancy, nursing following mammary gland involution), some are used by migratory immune cells to enable tissue-safe migration, some are overexpressed while organ repair after trauma (for example, skin healing or bone re-growth). All those natural processes require conditions named below:
(1) Re-structure (rearrange) extracellular matrix, especially rigid collagen scaffold, in order to make tissues soft, plastic, expandable stretchable and penetrable for cells, new-growing vessels, etc.,
(2) Maintain level of architectural integrity for affected tissues such, that it would allow the tissue to perform it's function while extracellular matrix is softened and rearranged. That would include, for example, cohesiveness of ligaments, intact structure of ducts within secreting organs, integrity of nerve axon bundles.

Expression and activity of tissue-remodeling enzymes, such as matrix metalloproteinases (MMPs), is part of natural, healthy self-maintenance of organism. Biological reason, therefore, proves safety and efficiency of such enzymes.

In mammalian (including human) organism such Tissue-Remodeling Enzymes would include a well-known family of matrix metalloproteinases (known as MMPs), and also less known families, such as: ADAMs, ADAMTSs, matriptases, collagenolytic cathepsins, collagenolytic members of kallikrein family and other endopeptidises, that possess protelytic activity towards extracellular matrix components that provide tissues stiffness.Some of those enzymes are proteolytic towards interstitial collagens (they are named collagenases), some towards collagen IV, some towards other extracellular matrix components. For example, native collagenases (proteolytical enzymes that are naturally responsible for digesting collagen structures in tissues during remodeling), would include gelatinase A (EC 3.4.24.24), neutrophil collagenase (EC 3.4.24.34), gelatinase B (EC 3.4.24.35), interstitial collagenase (EC 3.4.24.7), membrane-type matrix metalloproteinase-1 (EC 3.4.24.80), matrix metalloproteinase-11 (EC 3.4.24.B3), matrix metalloproteinase-13 (EC 3.4.24.B4), matriptase (EC 3.4.21.109), leukocyte elastase (EC 3.4.21.37), kallikrein 14 (EC 3.4.21.B45), cathepsin B (EC 3.4.22.1), cathepsin L (EC 3.4.22.15), cathepsin S (EC 3.4.22.27), cathepsin K (EC 3.4.22.38), meprin A (EC 3.4.24.18), matrilysin (EC 3.4.24.23). We include the list of individual TREZ enzymes in **Table 1.**

Since Tissue-Remodeling Enzymes have evolved to remodel tissues without harming them they have evolutionary developed properties that provide advantage over aggressive collagenases in such applications that require to maintain functional integrity within treated tissues.

Evolutionary-based functional difference between aggressive and tissue-remodeling collagenolytic and extracellular matrix-remodeling enzymes is reviewed in **Table 2.** We compare (1) bacterial, (2) digestive, (3) tissue-remodeling collagenolytic enzymes by set of criteria of biological reason for safety for mammalian tissues. We call "producing organism" such organism that has evolutionary developed the collagenolytic enzyme; we call "target tissue" the tissue that is biologically intended targed for the enzyme.

### Functional integrity of permanent tissues

We introduce two key definitions: functional integrity and permanent tissues.

### Biological function is different for proteolytical remodeling of permanent and temporary tissues

Tissue of our organisms can be divided into permanent and temporary. Permanent tissues maintenance is essential for (1) organism health and (2) survival of species, to which organism belongs. Examples of permanent tissues are: liver, kidney, pancreas, heart, muscle, nerve system, circulatory system, skin, tendon, cartilage. Lack or deficiency of permanent tissues is a disease (examples: lack of skin is wound or ulcer, lack of vasculature causes ischemia followed by necrosis, lack of liver or kidney causes systemic failure). We include reproductive organs (such as ovaries, uterus, testis, penis) because they are necessary for survival of the species, therefore, are permanent part of healthy organism.

Temporary tissues, such as fibrin clot, scar tissue or fat, are, as the definition implies, created to serve a temporary function (wound healing, blood clotting, energy storage) and intended later to be degraded by organism itself.

### Functional integrity of permanent tissues

Definition: Functional integrity is level of tissue cohesiveness such that maintains the architecture integrity level sufficient for tissue to perform it's function.

For permanent organ or tissue to be functional (examples: glucose-dependent insulin secretion by pancreatic islets, for muscle to contract upon signal from nerves, for tendon to transmit muscle contraction effort to skeleton), several integrity requirements have to be fulfilled:

### 1. Maintain tissue structure and compartmentalization

Permanent tissues evolved with a particular structure, tissue organization and compartmentalization to serve a biological purpose. For example, liver's vascular system, hepatocytes and immune system cells are strictly compartmentalized. In pancreatic islet insulin producing beta cells and vascular endothelial cells are proximal, but separated by a basement membrane. This arrangement is needed for a proper function of beta cells. In tendon the collagen fibrils are strictly organized to provide high level of elasticity and durability. Cohesion between collagen fibrils is essential for tendon durability and stress-resistance.

Blood-brain, blood-testis and blood-pancreatic barriers are examples of how compartmentalization is important. If these barriers fail, the immune system attacks brain, testis or pancreatic islets and destroys their function.

### 2. Maintenance of a functional cohesion between neighboring tissues

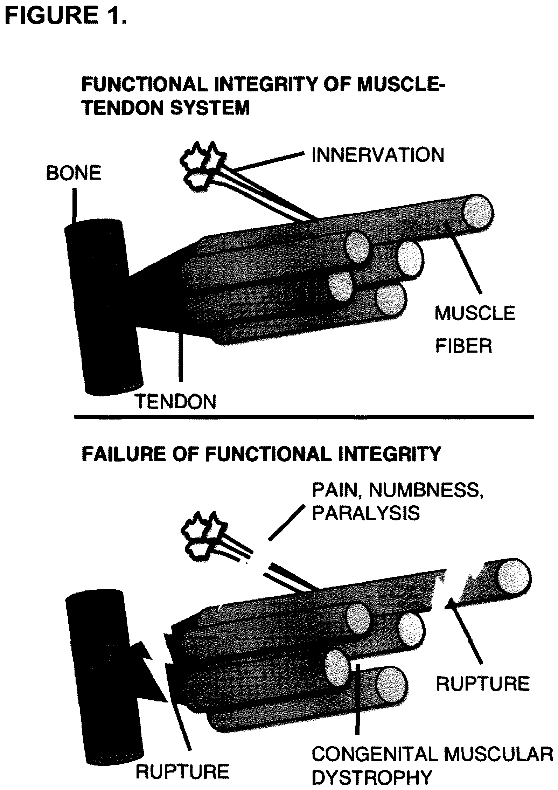

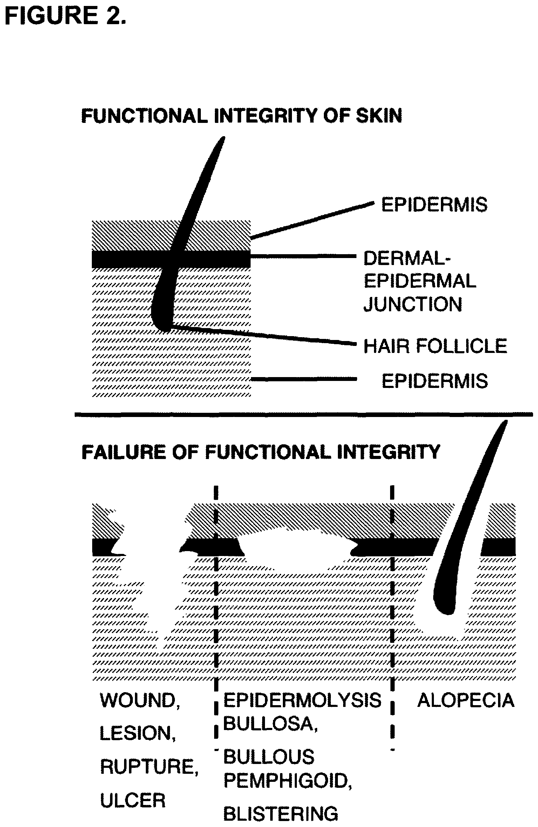

Functional integrity refers not only to a cohesion within tissue, but also how it connects to other tissues. **Figures 1****,** **2** and **Table 3** illustrate level required for functional integrity on example of muscle and skin. For example, if muscle maintains integrity and tendon maintains integrity, but muscle fails to connect tendon, then the whole muscle-tendon system fails to serve a biological function. If muscle fibers maintains integrity, but innervation within muscle is ruptured, the muscle will not able to react to neuronal signals and would fail the function. If skin dermis is detached from skin epidermis, it will cause blistering (example: epidermolysis bullosa, bullous pemphigoid, blistering)

### 3. Maintenance of cohesion between cells and their local niches

Many cell types can function properly only if they receive proper signals from local microenvironment (cell niche). It is important that cell keeps contact with particular neighboring cells (same type or different type), extracellular matrix around and maintains a proper focal orientation.

### Nerve-muscle-tendon system: functional integrity

Functional integrity of organ and surrounding tissues is level of integrity and architecture maintenance that is essential for organism to perform it's function. Let's see how it works on example of muscle and tendon.

Function of muscle is (1) to receive signal from motor neurons, (2) contract upon the signal, (3) pull the tendons, (4) the tendons would move the bones and the organism would move. Additionally, (5) the healthy, undamaged muscle should not send "pain" signals via sensory neurons.

If any of the parts is impaired, it would jeopardize the organism function (see Figure 1).

Therefore, the requirements for functional integrity for enzymatic treatment of tendon or muscle:
1. enzyme should not disrupt the nerves (motor or sensory)
2. enzyme should not maintain connection of muscle fibers to one another
3. enzyme should not disrupt muscle fibers as such
4. enzyme should not disrupt connection of muscle to tendon
5. enzyme should not disrupt the tendon (tendon must retain connection between contracting muscle and skeleton)

### Biological function of TREZ and functional integrity of permanent tissues

Nature needs TREZ for regenerative processes, for organs' development and growth during embryonic period, for tissue remodeling during reproduction, for migration of immune cells through connective tissues, for blood vessel growth in newly formed tissues. However, essential biological requirement is that during those processes all the permanent tissues must remain functional while treated by TREZ and afterwards; and, therefore must maintain functional integrity.

### Desired outcome: degradation or cohesive elastication of the tissue

The desired outcome of enzymatic treatment of tissue can differ from complete tissue degradation to subtle change of its mechanical properties.

### 1. Areas that consider tissue desintegration and loss of integrity as positive effect.

Desired outcome is degradation of extracellular matrix with release of cells or complete destruction of the tissue

Effect of tissue disruption or solubilization is desirable for purpose of the method. Highest speed and efficiency of tissue disruption is considered as criteria for method efficacy, not as hazard to safety and not as undesirable side effect.

### Areas that require maintenance of functional integrity while modifying the tissue

Desired outcome: to soften and elasticate the tissue while preserving a functional integrity of the treated tissue/organ and surrounding tissues/organ. Loss of functional integrity by aimed tissue or neighboring healthy tissues is considered a side effect.

### Examples:

2. Tissues for transplantation. The goal is to stretch them and adapt their shape for the recipient's lesion / wound shape.

### Results

We have observed that the application of TREZ with various tissue types had one or more effects. In this section we describe the nature and mechanics of these effects (in terms of tissue's structure, integrity, mechanoelastic and viscoelastic properties) as well as their evaluation methods. Applications, such as surgery, transplantation, regenerative medicine, reproductive medicine, veterinary etc, including when combination of several effects results in additional benefit, are disclosed in a separate section.

### Functional Integrity

We observed with several tissue types that treatment with TREZ modofies tissue's mechanical properties, while the tissue preserves its functional integrity.

### Cohesiveness

The tissue samples, treated by TREZ, remained in one piece, unlike samples treated by bacterial or crab collagenase which dissociated into incohesive parts.

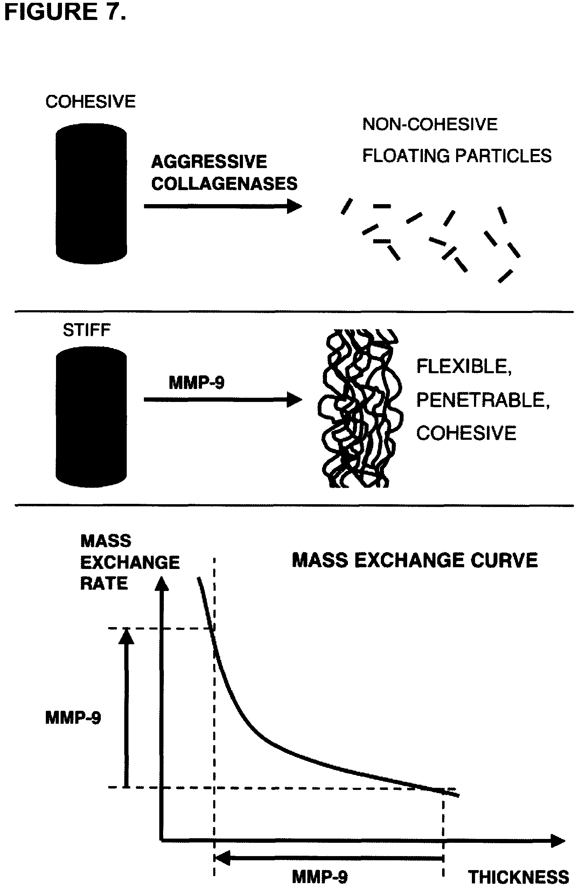

For example, MMP-9-treated tendon was split into individual, but interconnected fibers so that the tendon remains cohesive. Since the fibers remained intact and connected with each other and to the muscle, the functional integrity was preserved (**Figures 7****,** **8****,** **9**). In other example pancreas treated by MMP split into smaller lobules that are interconnected and have intact acini and intralobular ducts (**Figures 10****,****11**).

### Resistance to rupture

Large-scale magnification phase contrast microscopy revealed that cells within treated tissues remain cohesive: no microscopic ruptures were observed to separate the cells from their niches. In fact, MMP-9 treatment even enhanced cohesion for cells within liver sample; treatment with MMP-9 rendered liver tissues less prone to cell detachment and to microscopic ruptures.

The cohesiveness of TREZ-treated islet at a cellular level was observed with a help of phase contrast microscope. The islet cells remained attached to their neighbors under increasing pressure load.

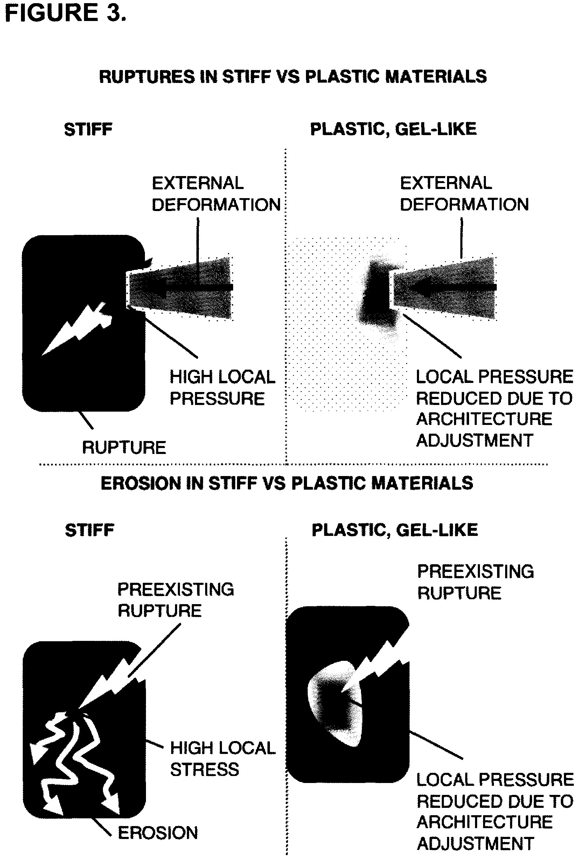

Resistance of TREZ-treated tissues to rupture and reduced brittleness (fragility) results from (1) increased plasticity and (2) reduced stiffness of TREZ-treated tissues. The mechanism of rupture- and erosion-resistance of TREZ-treated tissues is illustrated on **Figure 3****.**

### Functional cohesion between different tissue types

Not only it is important to maintain cohesion between cells within one tissue type, but also to maintain such cohesion between neighboring tissues that is important for their function (for example: lack of cohesion between neighboring muscle fibers may result in dystrophia, lack of cohesion between dermis and epidermis may result in blistering, lack of cohesion between hair and skin may result in alopecia).

### Cohesive under stress conditions

Tissues, treated by TREZ remained in one piece in the following conditions:
(1) suspended in buffer
(2) gently washed
(3) vigorously washed
(4) picked by forceps and hang in the air
(5) attached to adhesive surface and pulled by forceps
(6) subject to increasing pressure loads

### Modification of extracellular matrix: from stiff to plastic (gel-like)

### Permanent tissues are stiff and remember the original shape

Some permanent mammalian tissues can be described as stiff (bones) and most others as elastic (muscle, skin, tendons)

In order to change shapes of permanent tissues methods of cosmetic surgery and reconstructive surgery are used. Shape of tissues can be modified by scalpel or surgeon or by application of long-term pressure (such as dental braces to correct positioning of teeth).

Many applications of regenerative medicine would benefit from tissues rendered more plastic, for instance to adjust the grafted material to a particular shape.

Plastic materials easily change shape and adjust shape to form to which they are placed, adhere to surfaces better.

### Rupture and erosion of stiff materials vs plastic

As we discussed above, stiff and fragile tissues are prone to rupture and to erosion because little deformation is tolerated (see **Figure 3**). Once ruptured tissues become susceptible to further damage by erosion at even lower stress levels. The mechanisms of plasticity including implications for processes of brittleness (fragility), rupture and erosion are reviewed and illustrated in (Picu R.C. (2011), Hong W. et.al. (2008), Wang X. et.al. (2012)).

As treatment by TREZ makes tissues more plastic (gel-like), they are less prone to rupture and erosion. Microscopic ruptures within gels can heal (Picu R.C. (2011), Hong W. et.al. (2008), Wang X. et.al. (2012)).

### Permanent tissues, treated by tissue-remodeling enzymes (TREZ) modify from stiff materials into gel-like:

For many tissues we observed, that treatment with TREZ makes stiff, solid tissues more deformable, soft and adhesive, however they remain cohesive and thus maintain functional integrity.

### Creep assays to characterize plastic materials

### Creep assay is used to measure how stress causes gradual deformation.

The assay is carried out as follow. The tissue sample is placed on sensitive scale. A depth micrometer (Starrett 445MBZ-150RL Vernier Depth Gauge) is rigged on a stand above the sample. Micrometer screw applies a defined deformation to a tissue sample. The resulting stress is measured on the scale readout. Change of stress in time can be measured and recorded.

### Modification of extracellular matrix: from stiff to elastic

### TREZ-treatment allows to turn stiff tissues into stretchy

We observed that treatment with TREZ allows to improve stretch abilities of treated tissues:
1. to increase area of tissue
2. to spread thinner the tissue sample
3. to stretch more when force is applied
4. less pressure is required to cause same stretch deformation
5. part of tissue sample can be picked by pincet, stretched out far and observed (the tissue remains cohesive)

### Assays to qualify the effect

The best assay to evaluate stiffness vs stretch ability of tissues is building stress-strain curve. The curve allows to estimate
1) Lower stress in TREZ-treated samples required to achieve a specific deformation (strain)
(2) Increased deformation (strain) in TREZ-treated samples with equal stress However, we also used a set of correlating assays to qualify the effect.
   1) stress-strain curve (quantitative, multipoint assay)
   2) stress-relative area curve (qualitative, multipoint assay) qualitatively

   1) whether tissue remained intact when picked by forceps and pulled
   2) whether tissue remained intact when picked by two forceps from distal points and pulled apart
   3) whether tissue sample became gel-like and filled the inoculation loop
   4) whether it was possible to stretch the tissue on an adhesive surface

### Reduce brittleness (fragility) of fragile tissues: improve range of cohesive elastic deformation and reduce risks of rupture and erosion

### Tissues fragility: a risk for rupture and erosion

Many types of tissues loose their elasticity and become fragile as the person ages. The elastic tissue responds to stress with deformation and remains cohesive, while fragile tissue breaks (ruptures). Strain-stress curve can illustrate the phenomenon, where elastic tissue has lower stress-strain ratio, compared to stiff tissue. If the stress goes above the rupture barrier then the tissue breaks.

Important consequence of fragility is that, when once ruptured, it becomes more prone to damage (the rupture barrier declines). Plastic tissues are not as prone to erosion, on contrary, the rupture may even decrease with time, because highly plastic structures of gel-like structural organization have a limited capacity for self-healing (Picu R.C. (2011), Hong W. et.al. (2008), Wang X. et.al. (2012)).

### TREZ-treatment allows to reduce tissues brittleness (fragility)

Examples: MMP-9 treated and buffer-treated eye lens were subject to same force compression. While buffer-treated eye lens cracked, the MMP-9 treated eye lens resulted to external force with elastic deformation, but not with rupture.

### Assays to qualify the effect of TREZ

The best assay to evaluate stiffness vs stretch ability of tissues is building a stress-strain curve. The curve allows to estimate the limits of how far the tissue can be compressed (before and after TREZ treatment). We demonstrate for many tissues stress-strain curves which indicate that TREZ-treatment allows to increase limits of cohesive deformation.

Another assay is to apply same force to equivalent samples of tissue, treated by TREZ or by buffer (negative control). Start with low force and gradually increase, equal for both samples. As TREZ-treated sample reacts with deformation, the untreated sample (or one treated by buffer) would remain stiff until it suffers rupture at certain load. We demonstrate the effect on tissue samples of (1) pancreatic islets, (2) eye lense (half), (3) eye lens (whole).

### Improved adhesiveness

### Needs to improve tissue adhesiveness: surgery and transplantation

Normal tissues are not very adhesive to tissues or surfaces. However, tissues treated by TREZ become more gel-like and adhesive (1) to same type of tissue, (2) to different type of tissue, (3) to foreign surfaces.

However, increased adhesivity of mammalian tissues would be desired for medical applications, such as:
1) to allow better fit of transplant tissue to donor "wound" of complex shape
2) to allow wound borders fit closely, without gaps, in order to reduce risk of scarring
3) Adhesion would allow skin transplants to adjust to wound bottom of complex shape, thus enabling rapid exchange of gases, nutrients, waste products and enabling vascularization
4) Adhesion allows transplants or implants or wound borders to attach and hold firm. Not detach from each other by accident.
5) to improve embryo implantation to uterus

### Assays to evaluate adhesivity

Adhesivity is a consequence of tissue becoming more plastic. Besides plasticity assays there are qualitative assays to specifically evaluate adhesivity:
1) Does tissue sample adhere firmly to a inoculation loop? Can it be removed by forceps, or by washing with fluid?
2) Does tissue sample adhere firmly to a smooth surface (glass, plastic, etc)? Can it be detached by washing, gravity or centrifugation?
3) When tissue sample is placed on smooth surface, what is shape of meniscus between the tissue sample and the surface?
4) When the tissue is placed on same or other tissue, can it be easily detached by forceps or washing with fluid or centrifugation?

As we show for multiple types of tissue, treatment with TREZ significantly improved adhesive properties of tissues.

### Shape modification

### Need for shape modification

Possibility to change shape of permanent tissues would be highly desirable for (1) reconstructive surgery, (2) plastic surgery, (4) regenerative medicine, (5) transplantology and many other medical and veterinary applications (see Figure 12 for illustration). The natural stiffness of majority of permanent tissue allows them to maintain shape within range of physiological range for many years; however, for named medical applications it would be desirable to increase temporarily plasticity of tissue and mold it into desired shape.

However, it would be desirable that a method to modify shape of tissues would not cause ruptures, blistering, ulceration, detachment of teeth from their sockets, pain and numbness, caused by rupture of nerves.

### TREZ-treatment allows to modify shape and geometrical proportions of permanent tissues

We have shown for many permanent tissues (for example: lip tissues, penis tissues, skin dermis and other tissues) that treatment with TREZ allows them to easily modify and easily retain desired shape. Importantly, that when stretched, compressed or otherwise modified, the TREZ-treated tissue samples remained cohesive: no ruptures occurred.

### Assays to evaluate shape modification

The effect can be quantitatively described by the (1) plasticity stress-strain characteristics (described in previous chapter) and also by plots of: (2) area increase after temporary stress applied and removed and (3) reduction of tissue sample thickness after temporary stress applied and remove.

We also used several qualitative assays to describe ability of TREZ-treated tissue samples to change shape:
1) Is it possible to change shape of tissue sample by rearranging it on a coverglass?
2) Does the tissue sample attain the circular shape of inoculation loop, when placed within it?
3) Does the tissue sample mold into thin pipette tip, when placed inside it and subject to centrifugation?
4) Does the tissue sample retain it's original shape after 1 minute, 15 minutes, 2 hours, one day after temporary force was applied and removed?
5) Does the shape of tissue borders change from well-defined into diffuse and spherical?

### Improved permeability: function-wise architecture remodeling

### Need to improve permeability

Permeability is a key property to tissues viability and ability to expand.

Problem of tissues permeability appears in medicine and biotechnology when living tissues are separated from common blood circulation (in conditions such as: graft transplantation, islets for transplantation, ischemia, in vitro 3D tissue culturing). When thickness of tissue exceeds hundreds of microns and intrinsic blood circulation fails, the rate of gases, nutrients and waste products is reduced for cells within the tissue. Thickness of tissue lacking intrinsic blood circulation is critical for survival of cells within the tissue (Lehmann, Zuellig et al. 2007).

Effect of TREZ on tendon is shown on **Figures 7****,** **8** **and** **9****.**

Effect of TREZ on pancreas is shown on **Figures 10** **and** **11****.**

### Assays to evaluate permeability

Permeability is increased with (1) reduced thickness of individual components comprising tissue, (2) reduced stiffness and increased plasticity, that enables fluid flows within the tissue. On example of tendon and pancreas we can see such remodeling of tissue that allows to reduce thickness of individual components comprising the tissue many times. For many permanent tissues we demonstrated that TREZ-treatment allows to reduce thickness of the tissue sample significantly more, compared to tissues not treated by TREZ. Increased plasticity and decreased stiffness was quantitated and demonstrated for many permanent tissues.

### Practical applications

In this section we describe how TREZ effect on permanent tissues may be used to solve particular needs of plastic surgery, regenerative medicine, transplantation, reproductive medicine, wound debridement, in vitro tissue culture, and other practical applications.

### Transplantation, surgery

### Needs in transplantation

There in a number of needs that we aim to address by using TREZ on permanent tissues while maintaining functional integrity (see **Figure 12** for illustration):
1) Shortage of transplant material, therefore, need to stretch or expand
2) Need to reduce risks for transplant rejection; which includes: maintain viability of transplanted tissue and need to keep transplanted tissue adherent to host tissue
3) Need to maintain exchange of gases, nutrients and waste products (means: reduce risk for ischemia and necrosis) within transplanted organ; especially in case of grafts and islet transplantation
4) Need for host cells permeation, vascularization, innervation of transplant (dependent of tissue type)
5) Reduce risk of scarring
6) Reduce risk of pain or numbness or paralysis

### Skin transplantation

There is need for skin transplantation, for example, after extensive burns. Autologous skin would be best solution, however, (1) transplant material is limited, (2) there is problem how transplanted skin graft would remain viable until it is re-vascularized on the new place.

As we show, application of TREZ (MMP-9 in our example) to tissues (including skin dermis) allows to soften, stretch, adjust shape, improve adherent abilities, while maintaining integrity. We also demonstrate same formulation of TREZ does not disrupt integrity of nerve bundle.

Consequences for skin transplantation could be:
(1) to stretch and expand dermis to further extent compared to non-treated tissues and remain whole. Thus, less donor material is needed to cover the wound area
(2) to reduce thickness of dermis tissue sample, thus improving mass exchange between transplant and host tissue
(3) to make dermis more plastic: allow it to adjust complex shape of wound borders, reducing risks or scarring
(4) to make dermis more plastic: allow it to adjust to complex shape of wound bottom, thus allowing close fit (no gaps between transplant and host) and improving mass-exchange, reducing risks for ischemia within transplant, and improving possibilities for vascularization and re-innervation of transplant.

We demonstrate experimental effect of MMP-9 to reduce stiffness, increase plasticity and adhesiveness of treated skin dermis (see **Figure 17**; quantitative evaluation of the effect)

### Tissue fusion (cartilage and other soft tissues)

Patents US 8,323,642 B2 and US 20080145357 A1 by Story et.al. "Tissue fusion method using collagenase for repair of soft tissues" addresses the need of tissues fusion on example of healing an artificial lesion within knee meniscus.

Authors use bacterial collagenase in order to release cells from collagenase-digested tissues adjacent to the injury site, thus migration of released cells would allow closure of lesion.

We suggest, that TREZ-softening of transplant and/or host tissue suffering lesion would aid the fusion process:
1) allow to maintain infrastructure, architecture and functional integrity of TREZ-treated tissue
2) allow to fit transplant to host tissues without gaps
3) allow to extend borders of lesion in order to reduce size of lesion or even to merge the borders
4) allow adhesion of connected tissue borders that allows to reduce risk of accidental opening of lesion.

We demonstrate experimental effect of MMP-9 to reduce stiffness, increase plasticity and adhesiveness of treated cartilage (see **Figure 18**; quantitative evaluation of the effect). Effects for other soft tissues are shown on **Figures 13-24****.**

### Islet transplantation

Islet transplantation is extensively discussed below.

### Expanding tissues for transplantation

There is a need to increase amount of transplanted material without loss of functional structure of it.

Most organs of human organism have strong capacity to expand. Adult stem cells can undergo numerous divisions and maintain ability (1) to proliferate, (2) to differentiate into functional cell types. Not only stem cells, but also differentiated adult cells have high capacity for proliferation, for example: pancreatic insulin-producing beta cells, vascular endothelial cells, fibroblasts, keratinocytes, hepatocytes.

However, the adult organs have very limited capacity to expand (much lower, compared to ability of cells to proliferate). In healthy organism limitation of organ growth by extracellular matrix stiffness is a natural mechanism to restrict size of our internal organs

In medicine there are situations when expanding organs, or tissues, or islets many-fold would be beneficial

Natural stiffness of extracellular matrix in adult organ limits ability of organs to expand. However, in embryonic state the extracellular matrix of organs is soft and allows the organs to expand rapidly without loss of function or architecture. We suggest that TREZ-softening of organs, tissues or islets aimed for transplantation would allow adult organs to expand in the way similar to expansion of embryonic organs.

We shall review several examples:

### Surgery: flap technique

Flap techniques allow to increase amount of autologous tissues for transplantation. The advantage of method is that tissue (while growing) is connected to blood circulation, thus reducing risks of ischemia.

It would be beneficial if tissues aimed for flap replacement would grow fast enough and in amounts sufficient to cover the wound/lesion.

We suggest that use of TREZ on tissues aimed for flap technique would allow:
1) the tissue to expand more rapidly (thus reducing time for painful flap therapy and hospital stay)
2) the tissue to expand (by organ proliferation) to larger size (thus, if lesion is large and flap material is scarce - like in lip reconstructive surgery after tumor removal), material could be expanded sufficiently to cover whole lesion
3) the tissue spreading area can be expanded also by stretching the softened tissue sample

### Liver

Living donor liver transplantation is a method to cure patients with end stage liver disease, such as cirrhosis and/or hepatocellular carcinoma (caused, for example, by hepatitis C or B infection). The liver is organ highly capable of regeneration; hepatocytes have high proliferation capacity.

We suggest that TREZ-softening of liver could be used to allow (1) the transplanted liver lobe and (2) the liver lobes left in the donor to expand to healthy amount of liver tissue and have no restriction to newly formed liver architecture caused by extracellular matrix stiffness.

We demonstrate experimental effect of MMP-9 on treated liver tissues (see **Figures 19****,** **20**).

### Plastic surgery/ reconstructive surgery

Needs of plastic surgery and reconstructive surgery are:
1) to change the shapes of tissues (example: shape of nose, jaw, ear, facial tissues)
2) in case of cutting: to merge the lesion borders without gaps to avoid scarring
3) in case of injecting bioactive materials or scaffolds (like: collagen, hyaluronic acid, golden implants): to distribute the injected materials evenly, without bulges
4) to change positioning of organs/tissues (for example: teeth positioning in dentistry)

Ability of TREZ to soften the tissues without loss of functional integrity allows to serve those needs and to reduce risks of (1) tissue blistering or disruption, (2) complete loss of structure within treated tissue or neighboring tissues, (3) damage the nerve structure (thus causing pain, numbness or loss of motility).

We suggest some examples of plastic surgery that can benefit from applying TREZ to tissues (but not limited to those named below):
We demonstrate experimental effect of MMP-9 on a wide range of tissues, including tissues of lip, cartilage of ear and nose, skin, penis, female reproductive organs on **Figures 13-24****.**

### Islet transplantation

Donor-derived insulin-producing beta islets are successfully used for treatment Diabetes type I. Edmonton Protocol, currently used for treatment of diabetes patients, involves isolating islets from a cadaveric donor pancreas using a bacterial collagenolytic enzymes and injecting them into patient's portal vein aiming for attachment and hosting within patients liver. Several cadaveric donors are required to treat one patient. Efficiency of transplanted islets are several years.

There are several needs in area of islet transplantation into diabetic patients:
(1) Lack of human donor-derived islet material to provide help to all Diabetes type I patients.
(2) As several donors are required for transplantation, it is difficult to find all donors with same histocompatibility complex as the patient, therefore, problems with immune rejection and need for immunodepressants
(3) Human islets, due to size, after being extracted from pancreas, suffer ischemia due to size (in vitro storage of islets prior to transplantation)
(4) Poor survival rate of islets post-transplantation. Islets have problem with re-vascularization and re-innervation.
(5) Large size of human islets limits their ability to survive transplantation and function within patient after transplantation.

Using whole islets for transplantation has several advantages compared to transplantation of single beta-cells or beta-cell progenitors:
(1) pancreatic islet is a complex microorgan with function-wise cell compartmentalization
(2) architecture of pancreatic islets allows exchange of gases, nutrients and waste products between the endocrine cells within islet and blood circulation
(3) contact with blood circulation allows beta cells to detect level of glucose in blood and release insulin in glucose-dependent manner
(4) beta cells within islet form a syncytium structure, which enables all the beta cells within one islet to act in synergy.
(5) contact of insulin-producing beta cells with vascular endothelial cells, allows both of them to maintain functional phenotype

We have demonstrated that TREZ-treatment of pancreatic islets can allow to soften them and spread them thinner while maintaining functional integrity (without causing breakage (ruptures): see **Figure 23**). Also we have shown, that TREZ-treated islets become more adhesive: they can merge with tissues without gaps, hold attached and contact flat or complex surfaces without gaps. Such effect could be beneficial for the field of islet transplantation in following ways (but not limited to those):

### Improve exchange of gases, nutrients and waste products in donor-derived islets (in vitro)

Human islets, when derived from donor pancreases, suffer from insufficient exchange of gases, nutrients and waste products. In vivo the islets are surrounded by dense capsule, size of human islets varies within one to several hundred micrometers and mass exchange of endocrine cells within islets is enabled due to massive vascularization within the islet (a network of capillaries goes through each islet). After islets are detached from circulatory system, the inner capillaries of islet stop to serve function of mass exchange, and the only source of gases, nutrients and waste products is outside surrounding (in vitro culture medium or in vivo patient's bloodstream where the islets are injected).

Size of islets becomes a major problem in islet transplantation. (Lehmann, Zuellig et al. 2007) demonstrate that survival and post-transplantation function of human islets is in inverse proportion with the islet size.

We suggest that using TREZ on islets could aid the problem of size limitation on islet survival in following ways:
(1) modify shape of the islets, making them more flat (thus, improving accessibility of external molecules to the cells of the islet). Stretching of islets can be performed (for example): (1) by compression, (2) by adhesion to highly adhesive surfaces, (3) by centrifugation.
(2) allow to mechanically split the islets without ruptures into several cohesive parts, each maintaining local infrastructure (for example: by spreading and mechanically cutting into several pieces; by softening and mechanically splitting by sharp meshes, etc)
(3) by adhering the softened islets to adhesive surfaces, thus, reducing size of the islets.
(4) As islets become more soft and flexible, mass exchange can be enabled via flows of fluid, in the dynamic environment (for example: if the islet culture system is shaking or rotating or other way to enforce dynamic fluid flows around the islets)

Advantage of TREZ-softening is that modification of physical and geometrical properties of islet (softening, stretching, improved adhesivity, mechanically cut after softening) can be performed within few hours, before ischemia starts to affect the viability and healthy function of the islet cells.

### Improve exchange of gases, nutrients and waste products in patient-transplanted islets (in vivo)

Islets, after being transplanted into the patient, face the same problem of insufficient mass exchange rate. It takes over several days till islets may restore inner vascular flow connected to the patient blood circulation. In current islet transplantation protocols there is a problem, that part of islets fail to survive and function within the patient body.

We suggest that use of TREZ could improve survival of transplanted islets by the same means as described for in vitro survival.

Additionally, we suggest, that as TREZ treatment makes the islets more adhesive and gel-like, that property may allow the islets to adhere better to patient's tissues (without gaps), therefore, reducing risk of rejection and improving re-vascularization of transplanted islets.

### Improve banking (reducing size of islets)

There is a need to have banking of donor-derived islets. The islets can be frozen and stored in liquid nitrogen, however, size of islets sets limitations on the procedure (freezing and thawing whole islets is a more complicated compared to single cells). Reducing size of islets (or thickness) could reduce the risks and complications of islets banking.

Islet size can be reduced by using TREZ as described in previous chapters.

### Improve islet in vitro culture

Improving mass exchange within derived islets may be beneficial also for long-term in vitro cultures, for example (1) for scientists, working to understand islet function, (2) R&D specialists, working on ability to expand islets in vitro, (3) in drug screening, islet culture being a target for anti-diabetes drug candidates, (4) in drug screening, islet culture used to study side effects of potential drug candidates

### Enable islet expansion in vitro

Possibility to expand insulin-producing pancreatic islets in vitro is a long-wanted need in islet transplantation and diabetes therapy. If islets could be expanded in vitro, (1) less of allogenic cadaveric donor material (pancreases) would be needed for transplatation, (2) it would be possible to arrange banking of all histocompatibility donor types, (3) it would allow to use a biopsy from living donor (for example: relative or histocompatible volunteer) to serve one patient instead of several whole cadaveric pancreases (as it is now).

We suggest that using TREZ-softening effect on islets could serve the purpose of islet expansion in vitro in following ways:
(1) Improve mass-exchange and, therefore, survival of the islets (as described above)
(2) Reducing thickness of the islet would make the cells within islet more accessible for hormones and growth factors, added to cell culture medium in order to enhance islet expansion and function
(3) Allow mechanical split of one large islet into several or multiple functionally-cohesive parts, each of size sufficiently small to allow healthy mass exchange and growth
(4) TREZ-softening of extracellular matrix within islet allows to reduce local stress in place of cell proliferation (effect, described in previous chapters). TREZ-treatment would allow to remove the tissue stiffness-resistance limitation on possibility of islet and beta cells to expand.
(5) Combination of TREZ-treatment and mechanical passaging techniques would allow to split (repeatedly) the expanded islets without disrupting the local cell niche contacts.

We suggest that described TREZ-softening techniques could be compatible with adhesive, 2D or floating, 3D culturing systems of pancreatic islets.

### Enable islet passaging while maintaining functional integrity.

As reviewed above

### Tissue fusion

Demands of tissue fusion procedure and advantages of TREZ-softening of tissues are described in previous chapter (1).

Including such applications as (but not limited to those named below):
- tissues fusion in autotransplantation
- tissue fusion in allotransplantation
- tissue fusion in plastic surgery or reconstructive surgery
- cartilage tissue fusion to fill cartilage lesions and cracks

We demonstrate experimental MMP-9 effect on a wide range of soft tissues: see **Figures 13-24****.**

### Reduce risk of rupture and erosion

Previously we described how TREZ-softening of tissues would make tissue less prone to rupture and/or erosion. The effect can be used in many aspects of surgery, treating fragile skin prone to rupture and erosion, etc.

Use of TREZ could aid to serve following needs:

### Reduce risk of rupture or erosion

Living tissues sometimes suffer risk of accidental deformation, compression, tension that can cause high local stress and, therefore, local microscopic ruptures. In fragile tissues microscopic ruptures may undergo subsequent erosion and result in major ruptures.

As TREZ-treatment renders tissues more soft and gel-like, it would allow (1) to reduce local stress, (2) reduce risk of microscopic ruptures, (3) reduce risk of erosion for already occurring microscopic ruptures.

### Heal rupture or erosion

If tissues already suffer rupture or erosion, use of TREZ on ruptured tissue could (1) enhance ability of rupture to heal partially or completely and (2) reduce risk of increasing rupture as a result of accidental stress. The mechanisms are described above and are similar to ones described for "reducing risk or scarring" and "tissue fusion".

We demonstrate experimental MMP-9 effect of reducing brittleness and therefore reducing risk of rupture for (1) pancreatic islets, (2) eye lens: see **Figures 23** **and** **24****.**

### Plastic or reconstructive surgery

Plastic or reconstructive surgery of bones can benefit from use of TREZ as described above.

### Regenerative medicine

Regenerative medicine offers opportunities of creating artificial tissues using (1a) autologous patient's cells (adult cells, stem cells, iPS cells or else) or (1b) allogenic donor compatible cells and (2a) artificial scaffolds, (2b) patient's own extracellular matrix, (2c) allogenic or xenogenic extracellular matrix. Another option is expanding tissues (autologous or donor) in vitro to increase amount of transplant material. In further chapters we shall describe potential benefits of using TREZ for softening of allogenic or xenogenic extracellular matrix material, devoid of cells.

### Ophtalmology

We demonstrate experimental MMP-9 effect of reduced eye lens brittleness: see **Figure 24****.**

### Corneal transplantation (expand tissue)

There is a need for corneal transplantation in case of corneal trauma (example: burns).

### Regenerative medicine

Regenerative medicine offers opportunities of replacing a degenerated organ with a functional one, artificially engineered, or expanded from healthy tissue, or generated from human cells, untreated or modified, differentiated or stem cells in vitro. It would be highly desirable to expand small parts of healthy tissue, that can be taken as biopsy, into large ones for transplantation purposes. Possibility to expand in vitro whole tissues or organ parts instead of single cells could potentially allow to expand the functional organ of organized complexity (for example: podocyte cell culture expanded in vitro would not possess the functional organization of whole kidney). Therefore, methods to expand whole tissues would be highly desirable. However, the tissue proliferation ability is much lower compared to proliferation to single cell cultures comprising same tissue. Apparently, stiffness of tissue and low plasticity of it set strict limitation on possibility fo tissue to expand (that is how mammalian organisms restrain size of internal organs).

We suggest that ability of TREZ- treated tissues to reduce stiffness, increase plasticity and permeability would allow to improve techniques of regenerative medicine:
1) expand tissues by overcoming limitations on tissue expansive growth, set by natural stiffness of extracellular matrix
2) allow better permeability of tissues, therefore, improving possibility to grow tissues in vitro
3) allow better penetration of cells into decellularized matrices, used for purposes of regenerative medicine (reviewed further)
4) improve general methods of surgery, transplantology, reconstructive surgery that are part of regenerative medicine technique

### Reduce risk of scarring

Proteolytical enzymes are traditionally used to treat scars caused by (1) acne, (2) dermatological pathologies, (3) fragility or sensitivity of skin. The enzymes are traditionally used to be applied to scars and dissolve them.

We suggest that use of TREZ can serve the same need, but in a different method. The TREZ can be applied to skin suffering fragility, rupture, erosion, lesion (caused by acne or other pathology) in order to (1) reduce risk of rupture and erosion, (2) reduce risk of scarring by the mechanisms we discussed in previous sections.

### Culturing tissue explants in vitro

Including such applications as (but not limited to those named below):
- for following transplantation
- to study culture in vitro
- to culture and passage tissue in vitro while maintaining it's functional integrity

The use of TREZ for need to culture organotypic, artificial or other tissues or 3D structures like pancreatic islets in vitro was discussed in detailed in previous chapters, devoted to techniques of islet in vitro culture and regenerative medicine.

### Veterinary

Same methods that were discussed for human medicinal use, may be applied to treatment of animals.

### Non-living tissues: modifying physical properties

TREZ-treatment of tissues can also be applied to non-living tissues. Here we review several applications (though, not limited to those).

### Decellularized tissues and organs for transplantation purposes

Use of decellularized tissues and organs for transplantation purposes is a cost-efficient and highly beneficial method in transplantation and regenerative medicine. Extracellular matrix from allogenic or xenogenic donor can be transplanted and be inhabited by own cells of the patient or histocompatible donor (variants: stem cells, stem cells expanded in vitro, stem cells differentiated in vitro, adult cells, iPS cells, etc).

Advantages of transplanting the extracellular matrix (decellularized tissue or organ) from allogenic or xenogenic donor are:
1) Extracellular matrix, devoid of cells, will have no histocompatibility problems and immune rejection risk when transplanting from human to a human
2) Extracellular matrix is highly conservative between species. Therefore, transplantation of only extracellular matrix from animal donor (for example, pig) to human patient would have lower risks for rejection.
3) Extracellular matrix, decellularized, can be sterilized (for example: by irradiation, by autoclaving) in order to reduce risk of allogenic or xenogenic pathogens (for example: bacteria, viruses)
4) Extracellular matrix, decellularized , can be stored for long time (months, years), cold or frozen (+4C, -20C, -80C)

Decellularized tissues and organs that are developed, include:
1) Decellularized blood vessels
2) Decellularized liver
3) Decellularized kidney
4) Decellularized bone
5) Decellularized skin
6) Decellularized lung
7)

Reasons to use extracellular matrix of whole organ instead of soluble molecules mix:
1) Extracellular matrix of whole organ has nature-wise compartmentalization, architectural organization, which is beneficial for organ maintenance. When extracellular matrix is minced or solubilized, the complex structure is lost. The geometrical parameters are not the same, if soluble molecule mixture is delivered as gel or powder.
2) Organ stiffness: cell niche and differentiation. Native extracellular matrix has stiffness that defines cell behavior. For example, stiffness of extracellular matrix can define differentiation patterns of stem cells (Engler, Sweeney et al. 2007)
3) Organ stiffness: organ durability. Original bone extracellular matrix has mechanical properties (stiffness, stress-strain parameters, tension-wise and compression-wise durability) that are lost when demineralized extracellular matrix is introduced as powder

TREZ-treatment can be useful for extracellular matrix devoid of living cells. Whole extracellular matrix, devoid of cells, has same limitations for transplantation as a living transplant does. Treatment with TREZ prior to transplantation can improve following parameters:
1. Ability to stretch and adjust geometrical parameters (thickness, coverage area, complex shape)
2. Ability to adjust to complex shape of the defect (bode defect, skin wound, cartilage crack)
3. Ability to fit the complex shape without gaps, that would enhance:
   a. Cell permeation
   b. Vascularization (if needed)
   c. Exchange of gases, nutrients and waste products
4. Reduce risk of transplant detachment

### I. Experimental

### Materials and methods

### Tissue-Remodeling Enzymes

We have used human recombinant tissue-remodeling enzymes of matrix metalloproteinase (MMP) family and cathepsin family. To achieve properties close to those of native enzymes, we used (1) full-size enzymes in order to have all domains, (2) produced in mammalian (human or Chinese hamster) cells in order to attain proper glycosylation, (3) in lyophilized form in order to achieve enzyme high concentration, up to 1 mg/ml.

Activation was performed according to standard protocols: 4-aminophenylmercuric acetate (APMA) activation (0.5-1 mM) at neutral pH (pH7.5-8.0) in presence of calcium ions (1-10 mM) for enzymes of MMP family and disulfide bonds reduction by 5mM DTT (dithiothreitol) at acid pH (pH 5.0) for the cathepsins, followed by removal of activator. We would generally recommend to follow producer's instructions on enzyme reconstitution, activation, and storage.

Zymogens (full-size, pre-activated forms of enzymes) were restored from lyophilized form (according to producer's protocols) and transferred into activation buffers by means of dialysis. Buffer replacement (for example, to remove the activator) was performed by use of dialysis chambers with membrane MWCO (molecular weight cut-off) as 10,000 Da. Method of dialysis allows to change the buffer composition and maintain high concentration

Detailed procedures for MMP activation are reviewed in the book "Matrix Metalloproteinase Protocols" by Clark, Ian M, published 2001, Methods in Molecular Biology, ISBN 1592590462, Vol. 151 and numerous academic publications devoted to MMP studies.

### Tissue samples

Murine organs were placed in Hank's Balanced Salt Solution (HBSS) under dissection microscope, and uniform sections of tissue were excised by methods of microsurgery. Each section was dissected further into several uniform segments, that would serve as for experimental and for control group. As each animal has individual stiffness properties of organs (dependent on age and lifestyle), we aimed that tissue samples for experiment and control have to be derived from proximal tissues of same animal to attain maximal uniformity.

For reshaping and gel properties assays we generated the samples of tissues with well-defined angles of parallelepiped-like shape. In later assay the stiff tissues that behave like solid bodies would maintain the shape and angles, while gel-like tissues change to spherical shape with smoothened or undistinguishable angles.

Tissue samples were preliminary examined and photographed, prior to enzyme application.

Tissue types, used for our studies, were: skin, tendon (major limb tendons, including Achilles and minor finger tendons), cartilage (of nose and of ear), subcutaneous soft tissues of lip, penis foreskin, subcutaneous soft tissues of penis, liver, soft tissues of uterus wall, ovaries, pancreas, pancreatic insulin-producing islets of Langerhans, eye lens, nerve bundle (sciatic nerve, optic nerve), muscle, musculotendinous junction.

### Application of enzymes to tissue samples:

For in vitro assays: uniform tissue samples were generated, photographed and placed into plastic tubes, activated enzyme or control buffer solution being placed above. Tissue samples were gently rinsed by applied solution before incubation. Tissue samples were incubated at 37C at water bath (for non-living tissues) or cell culture incubator (for living tissues), time of incubation varying for each experiment (from 30 minutes to 4 days). During incubation tissue samples were gently rinsed by applied solution about 1-2 times per day. After treatment the samples were placed into HBSS solution devoid of enzymatic activity and subject to set of assays to qualify functional integrity maintenance, mechanoelastic and viscoelastic properties (such as: stiffness, plasticity, adhesivity, poroelasticity, fragility, cohesiveness, etc).

In vivo assay: thin segments of skin including dermis were generated in flap-like technique on anesthesized animals and activated enzyme vs control buffer were applied in form of solution were applied to each flap-like tissue segment in situ. The segments were rinsed by fresh solutions (of activated enzyme or buffer) several times per day for 2 days and solutions, after rinse, were kept applied to the treated segments. After treatment, the enzyme- and buffer-treated skin segments were excised and subject to set of assays to qualify functional integrity maintenance, mechanoelastic and viscoelastic properties (such as: stiffness, plasticity, adhesivity, poroelasticity, fragility, cohesiveness, etc).

### Functional integrity: qualitative evaluation

Each tissue sample was examined for maintenance of functional integrity after enzyme vs buffer treatment. Certain criteria of functional integrity are tissue-specific, such as: exocrine duct system integrity in pancreas, cohesion between muscle fibers in muscle, cohesiveness between two proximal ends of tendon. Tissue-specific criteria for functional integrity will be named for each tissue individually. However, general criteria are:
1. Does the tissue sample remain as one whole entity? Example: MMP-9 treated tendon stays as one whole entity, cohesiveness existing between two proximal ends. Clostridial collagenase-treated tendon is digested into microscopic floating particles, non-cohesive between each other.
2. Does the tissue maintain cohesion between neighboring cells?
3. Does the tissue undergo ruptures?

Functional integrity was observed (1) for tissue sample floating in buffer, (2) tissue sample being fixed and spread by means of a coverglass, (3) for tissue samples being subject to increased pressure loads.

We used different ranges of microscope magnification, from lowest to x40 in order to monitor macroscopic and microscopic (such as cell cohesion and microscopic ruptures) effects. We used several types of microscopy: (1) phase contrast on inverted microscope, (2) brightfield mode on dissection microscope, (3) dark field microscopy on dissection microscope. In phase contrast mode we variated settings for phase in order to distinguish between objects with similar transparity.

### Stress-strain characteristics for quantitative assay

Stress-strain characteristics of tissue are used in several assays and applications, such as: stiffness, creep assay, deformation under permanent load, residual deformation after temporary load.

Methods we used are a straightforward methods used for material stiffness evaluation in mechanics. However, we adjusted the mechanics methods to small size of our tissue specimen:

### Method 1: Apply stress to the tissue specimen and measure relative deformation.

The tissue specimen are placed in 24-well or 6-well tissue culture plate on coverglass and covered with HBSS buffer. The plate with specimen were placed under dissection microscope and photographed. Loads of progressive mass were applied to the specimen covered with coverglass; shape of load being thoroid in order to allow photograph the sample under the load. Coverglass placed upon the specimen were immersed in same buffer in order to avoid capillary effects that would distort the stress evaluation. The specimen were subject to increading loads of progressive mass. The specimen was photographed before and after the loads were applied; the surface area was measured by imaging program (in pixels, number being proportional to surface area).

Stress was evaluated as force (calculated as gravity force equal to load mass multiplied by g (strength of the gravitational field) and multiplied by calibration index to evaluate buoyant forced (caused by load being immersed in buffer)) being divided by the specimen area (to which the force is applied).

Strain was calculated as relative deformation of sample. Relative area is calculated difference in areas after load and prior to load, calibrated to initial area of specimen. Relative thickness of sample was estimated as reverse proportion of relative area of the specimen.

### Method 2: Apply deformation to the tissue specimen and monitor the resulting stress exhibited by specimen.

The tissue specimen were placed sensitive scale. A depth micrometer (Starrett 445MBZ-150RL Vernier Depth Gauge) is rigged on a stand above the sample. Micrometer screw applied a defined permanent deformation to a tissue sample. The resulting stress is measured on the scale readout and the change of stress in time was observed.

### Methods to assess plasticity

### Plasticity characteristics (residual strains after temporary stress applied)

The assay measures relative residual deformation (strain) after temporary stress applied. Permanent stress was applied for 2 minutes, afterwards removed and the specimen was allowed to restore previous shape for another 2 minutes. Residual deformation was measured by technique described above.

### Qualitative assays:

### See qualitative assays for reshaping

### Methods to assess stiffness

### Stress-strain characteristics (stiffness)

The assay measures relative deformation (strain) permanent stress applied as described before. Permanent load was applied to a tissue sample placed under dissection microscope, properties of load adjusted to allow photographing of the tissue sample under the load. Photographs were taken of tissue sample before loads, and following a series of increased loads. Stress and strain valued were calculated as described above.

### Methods to assess elastic vs viscous reaction to external stress/ deformation

### Qualitative: gel or elastic material

Tissue samples, treated by enzymes or buffer, can be qualitatively evaluated: do they behave like elastic material (like they used to before treatment) or like gels.

### Creep assay

We estimated viscoelastic properties of treated vs untreated tissues by applying permanent strain (as described above) and monitoring the kinetics of resulting stress within tissue sample. We use qualitative scale to represent the results of creep assay. MMP-treated tissues showed larger creep than untreated.

### Methods to assess adhesivity

We used a series of qualitative assays to assess adhesivity of tissue samples that were disclosed and discuss in previous chapters.

### Methods to assess cohesiveness

We used method of high-magnification microscopy to observe, whether individual cells within the treated tissues remain cohesive. If sample is moved, shaken or stretched under the microscope, we observe if individual cells in view field behave in cohesive manner and remain attached to the neighboring cells.

### Ability to attain different shape (reshaping)

We used a series of simple qualitative assays (also explained and discussed in previous chapters) to assess ability of the tissue sample to attain a different shape, for example:
1) ability of sample to attain a round shape within inoculation loop
2) ability of sample to attain conical shape, when placed within plastic pipette tip and subject to mild centrifugation, followed by relaxation period of 10 minutes
3) ability of sample to attain a different shape, when placed upon coverglass and moved by needles (stiff tissues move, but retain shape, while reshapable tissues easily attain different shapes).

Our qualitative observations correlate with quantitative results from plasticity assay.

### Permeability

Permeability depends on thickness of individual elements within tissue sample. Effects of increased permeability of reduced thickness can be evaluated for MMP-9 treated tendon and MMP-9 treated pancreas; as seen on **Figures 7-11****.**

### Experimental results

### Screening different enzymes and tissues

Preparations of human recombinant proteinases MMP-1, MMP-8, MMP-9, Cathepsin B and Cathepsin S (Sino Biologicals) were activated and transferred into working buffer of appropriate pH (neutral for MMP's and pH5.0 for cathepsins) and ionic additives (calcium ions for MMP's in concentration from 1 to 10 mM). The enzymes were diluted to concentrations of 0.03 mg/ml and applied to lip connective tissue samples and several other tissue types. Collagenase of Clostridium histolyticum (Roche) and crab collagenase (YO Proteins) served as controls for aggressive collagenolysis. Buffers used for dilution of MMP's and Cathepsins served as negative controls. The samples were observed for several days and evaluated by qualitative assays to (1) functional integrity maintenance, (2) stiffness reduction, (3) plasticity enhancement, (4) viscous reaction to external stress/deformation, (5) ability for reshaping.

According to assay, of MMP's and cathepsins group we selected a number of most representative experiments, that were reproduced later in different concentrations and time exposures and evaluated quantitatively in terms of stress-strain characteristics. Majority of experiments were performed with MMP-9, as it was, in our assay, most efficient to reduce stiffness and increase plasticity while maintaining functional integrity in vast range of tissues, from stiff constructive tissues (like tendon) to soft tissues (like liver or pancreatic islets). Vast majority of experiments was performed in vitro in order to reduce risk of accidental deformation of treated tissue samples, that may accidentally happen when applied to live animals in situ. Mouse tissues were used, unless otherwise specified.

### Lip connective tissue: reduced stiffness and increased plasticity after MMP-9 treatment

Subcutaneous tissues of lip contain highly innervated muscular and connective tissues. They are highly sensitive, responsible for mimics and mouth function. There are medical and cosmetical needs for modification of mechanic and shape characteristics of lip: (1) in reconstructive surgery (such as: cleft lip, lip tissues reconstruction after tumor removal), (2) in plastic surgery: to modify shape or size of lip, (3) in transplantology, (4) in cosmetic surgery, such as, insertion of implant materials (such as: collagen, silicone) into lip tissue in order to increase volume. Functional integrity requirement for such procedure would be (1) to maintain lip tissues cohesive (to avoid blisters and ulcers), (2) to avoid nerve rupture (such would cause pain, numbness, loss of motility)

Assay: Uniform samples of subcutaneous lip tissues were isolated from mouse lip after skin removal by methods of microsurgery and were incubated for 4 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution every day.

To assay compatibility with nerve integrity, uniform samples of sciatic nerve bundle were isolated and were treated by same enzymes formulation at same conditions.

Results: Tissue samples treated by MMP-9 (1) decreased stiffness, (2) increased plasticity, (3) improved adhesiveness while maintaining functional integrity. Tissue samples treated by collagenase of Clostridium histolyticum were dissociated into non-cohesive floating microscopic particles. Tissue samples treated by buffer (1) retained original stiffness and ability to maintain shape and (2) remained cohesive.

**Table 5** shows summary of qualitative assays.

**Figure 13** shows quantitative evaluation of stress-strain characteristics of lip tissue samples such as stiffness and plasticity, ability to increase area and to decrease thickness under stress.

**Figure 14** demonstrates effect of increased plascitity of MMP-9 treated lip tissue, compared to buffer-treated control.

**Figure 15** demonstrates effect of MMP-9 rendering lip tissue gel-like (adhesive, permeable, reshapable), compared to buffer-treated control.

### Lip connective tissue: reduced stiffness and increased plasticity after Cathepsin B treatment

Subcutaneous tissues of lip was treated by human recombinant Cathepsin B (concentration of 0.03 mg/ml; activated by 5 mM DTT at pH5.0 for 15 minutes at room temperature, which was subsequently removed by dialysis) vs buffer. The lip tissue was incubated with Cathepsin B vs buffer for 20 hours at 37C, being gently rinsed several times. Cathepsin B treated samples were of reduced stiffness and of increased plasticity compared to buffer-treated samples.

**Figure 16** shows quantitative evaluation of stress-strain characteristics of lip tissue samples such as stiffness and plasticity, ability to increase area and to decrease thickness under stress.

### Lip connective tissue: reduced stiffness and increased plasticity after MMP-8 treatment

Subcutaneous tissues of lip was treated by human recombinant MMP-8 (concentration of 0.03 mg/ml; activated by AMPA 0.5mM at pH8.0 at calcium ions concentration of 10mM for 2 hours at 37C) vs buffer. The lip tissue was incubated with MMP-8 vs buffer for three days at 37C, being gently rinsed 2 times per day. Qualitative evaluation shows that MMP-8 treated samples were less stiff and of increased plasticity compared to buffer-treated samples.

### Skin dermis: reduced stiffness and increased plasticity after MMP-9 treatment

Skin is a complex organ and it's proper function depends on cohesiveness within comprising tissue elements and between them (such as illustrated on **Figure 2** and **Table 3**).

There are medical and cosmetical needs for modification of mechanic and shape characteristics of skin, such as: (1) skin transplantation (to increase area, reduce thickness and adjust to shape of wound or lesion, (2) in reconstructive surgery, (3) in surgery: to reduce risks of scarring by making skin tissue more adhesive and plastic. Functional integrity requirement for such procedure would be (1) to maintain cohesiveness within dermis and within epidermis (to avoid ruptures and wounds), (2) maintain integrity of dermal-epidermal junction, (3) maintain coherence between hair and skin, to avoid alopecia, (4) to avoid nerve rupture (such would cause pain and numbness)

Assay: Uniform samples of whole skin (including dermis, epidermis and hair) were isolated by methods of microsurgery and were incubated for 2 days at 37°C in solution of: (1) MMP-9, 1 mg/ml, preactivated with 0.5mM APMA, (2) collagenase of Clostridium histolyticum, 1 mg/ml, (3) buffer solution. Samples were gently rinsed by applied solution every day and were subject to qualitative assay of integrity and mechanical characteristics.

Uniform samples of skin dermis (isolated from epidermis) were isolated by methods of microsurgery and were incubated for 2 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution every day and were subject to quantitative stress-strain and geometrical modification assessment, and for qualitative evaluation.

To assay compatibility with nerve integrity, uniform samples of sciatic nerve bundle were isolated and were treated by same enzymes formulation at same conditions.

Results: Tissue samples treated by MMP-9 (1) decreased stiffness, (2) increased plasticity, (3) improved adhesiveness while maintaining functional integrity. Tissue samples treated by collagenase of Clostridium histolyticum were dissociated whole tissue into non-cohesive floating microscopic particles (hair remained intact, but detached). Tissue samples treated by buffer (1) retained original stiffness and ability to maintain shape and (2) remained cohesive.

**Table 5** shows summary of qualitative assays.

**Figure 17** shows quantitative evaluation of stress-strain characteristics skin dermis samples such as stiffness and plasticity, ability to increase area and to decrease thickness under stress.

### Tendons: cohesive architecture remodeling, reduced stiffness and increased plasticity after MMP-9 treatment

Tendons are highly durable and highly elastic tissues. Each tendon consists of interconnected fibrils, tightly packed in order to ensure high durability. There are medical and cosmetical needs for modification of mechanic and shape characteristics of tendon: (1) to decrease stiffness in aged or traumatized tendon, (2) to enable tendon expansion (in case of short tendon), (3) to expand tendon tissue in vitro for medical and veterinary purposes. Functional integrity requirement for such tendon modifications would be: (1) to maintain connection between individual fibrils comprising tendon, so that network of interconnected fibrils behaves as whole, (2) to avoid local ruptures

Assay 1: Uniform samples of large limb tendon (Achilles or other) were isolated from mouse hind limbs by methods of microsurgery and were incubated for 1 day at 37°C in solution of: (1) MMP-9, 1 mg/ml, preactivated with 0.5mM APMA, (2) collagenase of Clostridium histolyticum, 1 mg/ml, (3) buffer solution. Samples were gently rinsed by applied solution.

Assay 2: Uniform samples of large limb tendon (Achilles or other) were isolated from mouse hind limbs by methods of microsurgery and were incubated for 3 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution, daily.

Assay 3: Uniform samples of finger tendon were isolated from mouse hind limbs by methods of microsurgery and were incubated for 1 day at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution.

Results: Tendons, treated by MMP-9 (1) split into interconnected, cohesive network of individual fibrils, (2) increased plasticity, (3) improved adhesiveness, (4) improved permeability while maintaining functional integrity. Tissue samples treated by collagenase of Clostridium histolyticum were completely dissociated into non-cohesive floating microscopic particles (assay 1) or partially dissociated into non-cohesive microscopic particles, while remains of tendon were partially ruptured. Tissue samples treated by buffer (1) retained original stiffness and ability to maintain shape and (2) remained cohesive.

**Table 5** shows summary of qualitative assays.

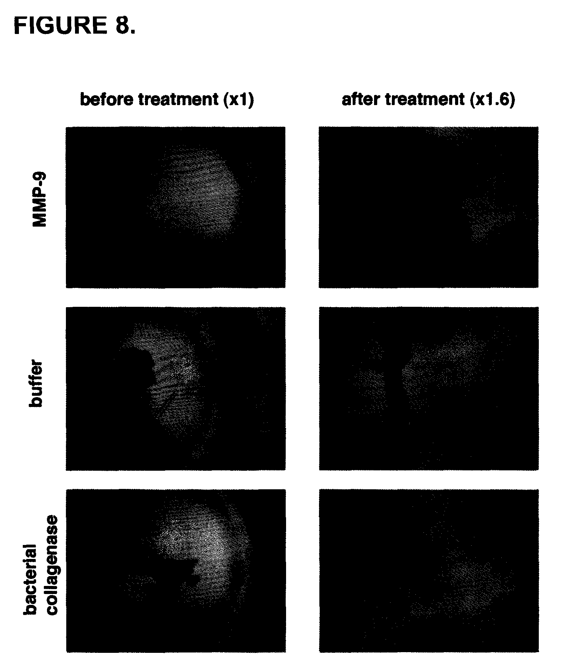

**Figures 8****,** **9** shows difference in tendon dissociation pattern, when treated by MMP-9 (cohesive remodeling) vs collagenase of Clostridium histolyticum (non-cohesive digestion). **Figure 7** illustrates the effect as a drawing.

### Cartilage (mouse and porcine): increased plasticity and reduced stiffness after MMP-9 treatment

Cartilage is a flexible connective tissue comprising joints between bones, rib cage, ear, nose, bronchial tubes and intervertebral discs. Modification of cartilage mechanical and shape propertied would be desirable (1) in plastic and reconstructive surgery (example: rhinoplasty), (2) for treatment of herniated intervertebrate discs, however, on condition that treated cartilage does not degenerate, rupture or blister.

Assay: Uniform samples of (1) nose cartilage (mouse) and (2) ear cartilage (mouse and porcine) were isolated as described above and incubated for 4 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution every day.

Results: Tissue samples treated by MMP-9 (1) decreased stiffness, (2) increased plasticity, (3) improved adhesiveness while maintaining functional integrity. Tissue samples treated by collagenase of Clostridium histolyticum were dissociated into non-cohesive floating microscopic particles. Tissue samples treated by buffer (1) retained original stiffness and ability to maintain shape and (2) remained cohesive.

**Table 5** shows summary of qualitative assays.

**Figure 18** shows quantitative evaluation of stress-strain characteristics of samples such as stiffness and plasticity, ability to increase area and to decrease thickness under stress.

### Penis: foreskin and subcutaneous connective tissues: reduced stiffness and increased plasticity after MMP-9 treatment

Modification of mechanoelastic, plastic and geometrical properties of penis tissues would be desirable in treatment of such conditions as: phimosis, Peyronie's disease, reconstructive, plastic and cosmetic surgery, however, on condition (1) that cohesion of penis tissues maintains and (2) innervation is not ruptured by the method.

Assay: Uniform samples of (1) penis foreskin and (2) subcutaneous connective tissues of penis were isolated by methods of microsurgery and incubated for 2 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution every day.

To assay compatibility with nerve integrity, uniform samples of optic nerve were isolated and were treated by same enzymes formulation at same conditions.

Results: Tissue samples treated by MMP-9 (1) decreased stiffness, (2) increased plasticity, (3) improved adhesiveness while remaining cohesive. Tissue samples treated by collagenase of Clostridium histolyticum were dissociated into non-cohesive floating microscopic particles. Tissue samples treated by buffer (1) retained original stiffness and shape and (2) remained cohesive.

**Table 5** shows summary of qualitative assays.

### Liver: reduced stiffness and increased plasticity after MMP-9 treatment

Modification of liver tissue stiffness would be desirable for expanding liver tissues for transplantation purposes, however, on condition that the treatment would maintain the complex composition of liver cells and compartments cohesive.

Assay: Uniform liver tissue samples incubated for 3 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution every day.

To assay compatibility with nerve integrity, uniform samples of optic nerve were isolated and were treated by same enzymes formulation at same conditions.

Results: Tissue samples treated by MMP-9 (1) decreased stiffness, (2) increased plasticity, (3) improved adhesiveness while maintaining functional integrity. Tissue samples treated by collagenase of Clostridium histolyticum were dissociated into non-cohesive floating microscopic particles. Tissue samples treated by buffer (1) retained original stiffness and ability to maintain shape and (2) remained cohesive. MMP-9 treated tissue allowed to maintain cells more cohesive within liver tissue sample, compared to buffer-treated tissue.

**Table 5** shows summary of qualitative assays.

**Figure 19** shows quantitative evaluation of stress-strain characteristics of samples such as stiffness and plasticity, ability to increase area and to decrease thickness under stress.

**Figure 20** illustrates the effect

### Pancreas: adhesive architecture remodeling improving permeability after MMP-9 treatment

Improving permeability of pancreatic tissue would be desirable for diabetes treatmens: (1) pancreas transplantation and (2) in vitro expansion of pancreatic tissue, however, on condition that tissue maintains functional integrity, which includes cohesion between exocrine cells and integrity of lumens and intralobular ducts, necessary for exocrine function.

Assay: Uniform samples of pancreas, approximately 1 mm in diameterm, were isolated from pancreas by methods of microsurgery and incubated for 1 day at 37°C in solution of: (1) MMP-9, 1 mg/ml, preactivated with 0.5mM APMA, (2) collagenase of Clostridium histolyticum, 1 mg/ml, (3) buffer solution. Samples were gently rinsed by applied solution.

Results: Tissue samples treated by MMP-9 have changed the architecture: solid piece of tissue separated into interconnected lobules in manner that maintained cohesion of cells within lobules and integrity of intralobular ducts. The lobules thickness was substantially lower compared to that of original tissue sample, thus, remodeling improved tissue permeability. Tissue samples treated by collagenase of Clostridium histolyticum were digested into non-cohesive floating microscopic particles. Tissue samples treated by buffer (1) retained original stiffness and shape and (2) remained as one solid part.

**Table 5** shows summary of qualitative assays.

**Figure 11** shows difference in pancrea dissociation pattern, when treated by MMP-9 (cohesive remodeling) vs collagenase of Clostridium histolyticum (non-cohesive digestion). **Figure 10** illustrates the effect as a drawing.

### Pancreatic insulin-producing islets of Langerhans: reduced stress-strain ratio and increased adhesivity after MMP-9 treatment

Ability to modify stiffness, plasticity, fragility, adhesivity and thickness of pancreatic islets would allow to solve a number of needs in islet transplantation for treatment of diabetes patients. However, it is highly desirable to maintain functional cohesion between cells within islet, as interaction between cells are important for proper islet function.

Assay: Islets of Langerhans were isolated according to standard protocols and incubated for 1 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed.

Results: Islets treated by MMP-9 (1) increased adhesivity, (2) increased plasticity and ability to reduce thickness, (3) increased cohesiveness between cells undergoing deformation. Tissue samples treated by collagenase of Clostridium histolyticum were dissociated into non-cohesive floating microscopic particles. Tissue samples treated by buffer (1) retained original stiffness and ability to maintain shape and (2) remained cohesive. MMP-9 treated islets could be deformed to high extent while cells remained cohesive and no ruptures occurred, compared to buffer-treated islets that ruptured under same deformation.

**Table 5** shows summary of qualitative assays.

**Figure 23** demonstrates effect of increased plasticity and increased cohesiveness within of MMP-9 treated pancreatic islets, compared to buffer-treated control that underwent rupture under same load.

### Eye lens: reduced stiffness and increased plasticity after MMP-9 treatment

A method to reduce stiffness and reduce fragility of eye lens would be desirable in ophthalmology, however, that such method would not rupture the lens and optic nerve.

Assay 1: Eye lenses were isolated from eyes of same healthy animal for MMP-9 and for buffer treatment to avoid potential differences in stiffness due to age and background. Eye lens for collagenase from Clostridium histoliticum was derived from animal of same age and condition. The eye lens samples were incubated for 4 days at 37°C in solution of: (1) MMP-9, 0.1 mg/ml, preactivated with 0.5mM APMA, (2) collagenase of Clostridium histolyticum, 0.1 mg/ml, (3) buffer solution. To assay compatibility with nerve integrity, uniform samples of optic nerve were isolated and were treated by same enzymes formulation at same conditions.

Results: Tissue samples treated by MMP-9 maintained functional integrity and were less less fragile: when pressure applied, the samples responded with elastic deformation, but not rupture. Tissue samples treated by buffer (1) retained original stiffness and shape and (2) responded to pressure by rupture, but not by elastic deformation.

Tissue samples treated by collagenase of Clostridium histolyticum were dissociated into non-cohesive floating microscopic particles.

**Table 5** shows summary of qualitative assays.

**Figure 24** illustrates effect of MMP-9 treated eye lens is less prone to rupture, compared to buffer-treated control.

### Nerve: maintained integrity and reduced stiffness after MMP-9 treatment

Many medical and veterinary application that involve use of proteolytical enzyme on tissues would benefit, if risk of nerve rupture was reduced (such reducing risks of pain or numbness). Integrity of nerve bundles in important for most enzymatic therapies.

Assay: Uniform samples of (1) sciatic nerve or (2) optic nerve were isolated by methods of microsurgery and were incubated for up to 4 days at 37°C in solution of: (1) MMP-9, 0.1 mg/ml, preactivated with 0.5mM APMA, (2) collagenase of Clostridium histolyticum, 0.1 mg/ml, (3) buffer solution. Samples were gently rinsed by applied solution daily.

The assay described hereby served as a control for several other tissues (see above)

Results: Tissue samples treated by MMP-9 (1) maintained integrity of single nerves and of whole nerve bundle (no ruptures were observed, (2) integrity on nerves maintained also under deformation, (3) stiffness (stress-strain ratio) of MMP-9 treated sample reduced, thus allowing increased range of cohesive nerve deformation. Tissue samples treated by collagenase of Clostridium histolyticum were dissociated into non-cohesive floating microscopic particles. Tissue samples treated by buffer (1) retained original stiffness and ability to maintain shape and (2) remained cohesive.

**Table 5** shows summary of qualitative assays.

### MMP-9 reduces stiffness of muscle.

Tissue samples: (1) muscle samples including muscle fibers wrapped in endomysium and perimysium were subject to qualitative stress-strain assay (deformation under load); (2) muscle samples including myotendonous junction were used for qualitative assessment and to control integrity of myotendonous junction; (3) optic nerve and sciatic nerves were used as a control: being subject to enzyme of same formulation (concentration, activity, buffer composition, etc) in order to control integrity of nerves.

Results: MMP-9 treatment for 3 days at 37C in concentration of 0.1 mg/ml allowed mild (though noticeable) reduction of muscle sample and increased plasticity (compared to buffer-treated control). Functional integrity of muscle, myotendonous junction and control nerve sample remained conserved: no rupture within muscle fibers, nerve fibers, endomysium, perimysium, myotendonous junctions observed and muscle fibers remain cohesive to each other within sample; also in applied pressure assay. Tissue samples treated by collagenase of Clostridium histolyticum were digested into non-cohesive floating microscopic particles (1) individual muscle fibers were digested into particles, (2) connection between neighboring fibers was lost, (3) myotendonious junction was digested. Tissue samples treated by buffer (1) retained original stiffness and ability to maintain shape and (2) remained cohesive.

**Table 5** shows summary of qualitative assays.

### Tables

**Table 1. List of individual enzymes belonging to group of tissue-remodeling enzymes (TREZ):**

| # | MMP | Alternative name(s) | EC# | UniProt# |
|---|---|---|---|---|
| 01 | MMP-1 | Interstitial collagenase; Fibroblast collagenase; Matrix metalloproteinase-1 | EC=3.4.24.7 | P03956 (MMP1_HUMAN) |
| 02 | MMP-2 | 72 kDa type IV collagenase, 72 kDa gelatinase, Gelatinase A, Matrix metalloproteinase-2 | EC=3.4.24.24 | P08253 (MMP2_HUMAN) |
| 03 | MMP-3 | Stromelysin-1, Matrix metalloproteinase-3, Transin-1 | EC=3.4.24.17 | P08254 (MMP3_HUMAN) |
| 04 | MMP-7 | Matrin, Matrix metalloproteinase-7, Pump-1 protease, Uterine metalloproteinase | EC=3.4.24.23 | P09237 (MMP7_HUMAN) |
| 05 | MMP-8 | Neutrophil collagenase, Matrix metalloproteinase-8, PMNL collagenase, PMNL-CL | EC=3.4.24.34 | P22894 (MMP8_HUMAN) |
| 06 | MMP-9 | Matrix metalloproteinase-9, 92 kDa gelatinase, 92 kDa type IV collagenase, Gelatinase B | EC=3.4.24.35 | P14780 (MMP9_HUMAN) |
| 07 | MMP-10 | Stromelysin-2 (SL-2), Matrix metalloproteinase-10, Transin-2 | EC=3.4.24.22 | P09238 (MMP10_HUMAN) |
| 08 | MMP-11 | Stromelysin-3 (SL-3), Matrix metalloproteinase-11 | | P24347 (MMP11_HUMAN) |
| 09 | MMP-12 | Macrophage metalloelastase, Matrix metalloproteinase-12 | EC=3.4.24.65 | P39900 (MMP12_HUMAN) |
| 10 | MMP-13 | Collagenase 3, Matrix metalloproteinase-13 | | P45452 (MMP13_HUMAN) |
| 11 | MMP-14, MT1-MMP | Matrix metalloproteinase-14, MMP-X1, Membrane-type matrix metalloproteinase 1v(MT-MMP 1, MTMMP1), Membrane-type-1 matrix metalloproteinase(MT1-MMP, MT1MMP) | EC=3.4.24.80 | P50281 (MMP14_HUMAN) |
| 12 | MMP-15 | Membrane-type matrix metalloproteinase 2 | | P51511 (MMP15_HUMAN) |
| 13 | MMP-16 | Membrane-type matrix metalloproteinase 3 | | P51512 (MMP16_HUMAN) |
| 14 | MMP-17 | Membrane-type matrix metalloproteinase 4 | | Q9ULZ9 (MMP17_HUMAN) |
| 15 | MMP-18 | Matrix metalloproteinase-18, Collagenase-4 (xCol4) | | 013065 (MMP18_XENLA) (Xenopus seq.) |
| 16 | MMP-19 | Matrix metalloproteinase RASI, Matrix metalloproteinase-18 | | Q99542 (MMP19_HUMAN) |
| 17 | MMP-20 | Enamel metalloproteinase, Enamelysin | | 060882 (MMP20_HUMAN) |
| 18 | MMP-21 | Matrix metalloproteinase-21 | | Q8N119 (MMP21_HUMAN) |
| 19 | MMP-23A | Femalysin, MIFR-1, MMP-22 | | 075900 (MMP23_HUMAN) |
| 20 | MMP-23B | Femalysin, MIFR-1, MMP-22 | | 075900 (MMP23_HUMAN) |
| 21 | MMP-24 | Membrane-type matrix metalloproteinase 5 | | Q9Y5R2 (MMP24_HUMAN) |
| 22 | MMP-25 | Leukolysin, Membrane-type matrix metalloproteinase 6 | | Q9NPA2 (MMP25_HUMAN) |
| 23 | MMP-26 | Endometase, Matrilysin-2 | | Q9NRE1 (MMP26_HUMAN) |
| 24 | MMP-27 | Matrix metalloproteinase-27 | | Q9H306 (MMP27_HUMAN) |
| 25 | MMP_28 | Epilysin | | Q9H239 (MMP28_HUMAN) |
| 26 | matriptase | | (EC 3.4.21.109) | |
| 27 | leukocyte elastase | Neutrophil elastase, Bone marrow serine protease, Elastase-2, Human leukocyte elastase (HLE), Medullasin, PMN elastase | (EC 3.4.21.37) | P08246 (ELNE_HUMAN) |
| 28 | kallikrein 14 | | (EC 3.4.21.B45) | Q9P0G3 (KLK14_HUMAN) |
| 29 | cathepsin B | APP secretase (APPS), Cathepsin B1 | (EC 3.4.22.1) | P07858 (CATB_HUMAN) |
| 30 | cathepsin L | | (EC 3.4.22.15) | |
| 31 | cathepsin S | | (EC 3.4.22.27) | |
| 32 | cathepsin K | | (EC 3.4.22.38) | |
| 33 | meprin A | | (EC 3.4.24.18) | |
| 34 | matrilysin | | (EC 3.4.24.23). | |
| 35 | Granzyme A | CTL tryptase, Cytotoxic T-lymphocyte proteinase 1, Fragmentin-1, Granzyme-1, Hanukkah factor | EC 3.4.21.78 | P12544 (GRAA_HUMAN) |
| 36 | Granzyme B | | EC 3.4.21.79 | |
| 37 | Granzyme H | | | P20718 (GRAH_HUMAN) |

Classification Sources: www.pubmed.gov, http://www.uniprot.org, http://enzyme.expasy.org

**Table 2. Evolutionary functional differences between aggressive (bacterial or digestive) collagenases and Tissue-Remodeling Enzymes (TREZ)**

| # | criteria | Bact. | Digest. | TREZ |
|---|---|---|---|---|
| 1 | Producing organism aims to apply enzyme on it's own tissues | NO | NO | YES |
| 2 | Producing organism aims to keep permanent mammalian tissues functional | NO | NO | YES |
| 3 | Producing organism aims to preserve the functional integrity and architecture of aimed tissue | NO | NO | YES |
| 4 | Producing organism aims to preserve cohesive the aimed tissue | NO | NO | YES |
| 5 | Producing organism aims collagenolysis for healthy purposes for aimed tissue (like: regeneration, expansion, development) | NO | NO | YES |
| 6 | Producing organism aims to preserve safe and functional the mammalian tissues neighboring to the aimed tissue | NO | NO | YES |
| 7 | Producing organism aims to preserve safe the nerves (axons) within and around the aimed tissue | NO | NO | YES |

**Table 3: Summary of functional integrity of muscle and skin**

| # | Tissue unit | Example of disease when integrity is failed |
|---|---|---|
| | **Functional integrity of muscle and tendon** | |
| 1 | Nerves | Numbness (sensory nerves), pain (sensory nerves), paralysis (motor nerves) |
| 2 | Muscle fiber wholeness | Muscle rupture, |
| 3 | Muscle fibers: connection between fibers | Congenital muscular dystrophy: mutation of collagen VI or merosin |
| 4 | Tendon: retain connection between muscle and sceleton | Tendon rupture |

| | **Functional integrity of skin** | |
|---|---|---|
| 1a | Wholeness of epidermis layer | Wound, ulcer, blister, |
| 1b | Wholeness of dermis layer | |
| 2 | Connection between dermis and epidermis (wholeness of dermal-epidermal junction) | Epidermolysis bullosa (mutations of genes: LAMA3, LAMB3, LAMC3) |
| 3 | Hair integration into skin | Alopecia |

**Table 4.**

| Mechanical characteristics (such as stiffness, stress-strain ratio, fragility, plasticity, etc.) and shape characteristics (such as: shape, thickness, positioning angle, area, etc) of mammalian permanent tissues can be modified by use of TREZ: translation of mechanical and shape characteristics into medical and cosmetical applications. | | | | |
|---|---|---|---|---|
| Diseases caused by pathological stiffness of organs and tissues: can be treated by using TREZ to reduce stiffness (stress-strain ration) back to normal | | | | |
| # | Disease | Evaluation criteria for disease and treatment efficacy | Mechanoelastic properties of tissue resulting the disease | How use of TREZ to modify the mechanoelastic characteristics can be used for disease treatment |
| 1. | Adhesive capsulitis (frozen shoulder), | Range of shoulder motility is limited. Degree of pathology is evaluated as: (1) rotational range of motions (ROMs) and (2) glenohumeral (GH) stiffness | Increased stiffness of connective tissues within shoulder, therefore,ROM is decreased in disease. (Lin, Hsu et al. 2008; Koh, Chung et al. 2012) | TREZ treatment allows to decrease stress-strain ratio, therefore range of strain (range of shoulder movements) can be increased within physiological limits of stress (force that does not cause pain or damage). See **Figure 4****.** |
| 2. | Dupuytren's contracture | Angle of finger movement; is limited by stiffened / shortened Dupuytren's cords. The range of finger bending angle is evaluation criteria (Hurst, Badalamente et al. 2009) | Increased stiffness of Dupuytren's cords / shortened shape of Dupuytren's cords. | TREZ treatment allows to decrease stress-strain characteristics of connective tissues; therefore, Dupuytren's cords can be either made more elastic, or be extended (shape modification). |
| 3. | Phimosis | Inability of penis foreskin to extend enough to allow erection. | Increased stiffness of penis foreskin | TREZ treatment allows to reduce stiffness of penis tissues, therefore, healthy range of foreskin stretching can be restored. See **Figure 5**. |
| 4 | Stiffened eye lens; poor accomodation | Accomodation range (range of distance to which an eye can focus) | Increased stiffness of eye lens | TREZ treatment allows to reduce stiffness (reduce stress-strain ration), therefore, the range of strain would increase, therefore, range of eye accommodation would increase |
| 5 | Stiffened eye lens; increased fragility | Stiffened eye reacts to external force not by elastic deformation, but by rupture (breakage) | Increased brittleness, increased stiffness (Hansen, Stitzel et al. 2003; Stitzel, | TREZ treatment allows to make eye lens more elastic and therefore, less fragile and less prone to rupture caused by external force |
| | | | Hansen et al. 2005) | |
| 6 | Liver fibrosis and liver cirrhosis | Stress-strain characteristics correlate with extent of liver fibrosis and cirrhosis. Increased stiffness of liver hinders ability of liver to regenerate. | Stiffness of liver is not just a consequence, but also part of pathogenesis (Georges, Hui et al. 2007). | TREZ can be used to reduce stiffness (reduce stress-strain ration) for liver tissues, therefore, allow regenerative processes and restore function |
| 7 | Pulmonary fibrosis | Ability of lung for elastic deformation within healthy range | Increased stiffness of extracellular matrix within lungs | TREZ can be used to reduce stress-strain ration, therefore, increase range of lung inflation |

| **Medical and cosmetical needs for increased plasticity: can be treated by using TREZ to reduce stiffnes, increase plasticity and adhesivity** | | | | |
|---|---|---|---|---|
| # | Medical procedure | Evaluation criteria for disease and treatment efficacy | Mechanoelastic properties of tissue resulting the disease | How use of TREZ to modify the mechanoelastic characteristics can be used for disease treatment |
| 8 | Plastic surgery (rhinoplasty, otoplasty, mammoplasty, etc) | Shape or organ (nose, ear, breasts, etc) that needs to be modified | Natural stiffness (stress-strain characteristics) of tissues make tissues "remember" original shape. | TREZ-treatment may be used to make tissues more plastic, therefore, more easy to attain desired shape. Use of temporary scaffold for intended shape would be beneficial (such as: dental braces for teeth or gold threads for facelift). |
| 9 | Reconstructive surgery for cleft lip. | Extent of cleft (shape, depth of cleft) | Stiffness and low adhesivity of untreated lip tissues | TREZ-treated lip tissues could (1) expand, (2) merge to cover the cleft |
| 10 | Incorrect positioning of teeth | Angle of incorrect tooth positioning | Connective tissues around tooth are too stiff to allow easy change of tooth positioning | Applying TREZ to connective tissues around tooth may allow to achieve better effect using dental braces. See **Figure 6****.** |
| 11 | Islet transplantation: increase survival rate | Size of islet | Untreated islets are too stiff to allow cohesive deformation into flat shape | TREZ treatment may make make islets more plastic and allow cohesive reshaping into flat structures |
| 12 | Transplantation: adhesion of transplanted tissue to host tissue | Gaps between transplanted tissue and host tissue | Natural stiffness and low adhesiveness of tissues may cause gaps between transplant and host tissues | TREZ treatment increases plasticity and adhesiveness of tissues. If transplant or host tissues are TREZ-treated, plasticity and adhesiveness would allow to reduce gaps. See **Figure 12****.** |
| 13 | Scarring in open surgery | Extent of scarring | Natural stiffness and low adhesiveness of tissues may cause gaps between lesion | TREZ treatment increases plasticity and adhesiveness of tissues: could be used to allow gapless fusion of lesion borders, thus, reducing |
| | | | borders | risk of scarring. |
| 14 | Wrinkles reduction in cosmetics | Depth and other geometrical proportions of wrinkles | Natural stiffness of skin dermis makes it "remember" original shape, i.e. wrinkles | Making skin dermis more plastic, shape of skin can be modified to reduce wrinkles. Use of durable scaffold (for example: gold threads) may be beneficial. |

**Table 5: MMP-9 ability to modify (1) stiffness, (2) plasticity, (3) adhesivity, (4) ability to reshape, (5) fragility in tissues compared to buffer-treated samples**

| | | Functional integrity | Stiffness | Plasticity | Adhesivity | Reshaping | Fragility |
|---|---|---|---|---|---|---|---|
| 1 | Lip | maintains | Decreased, highly | Increased, highly | Increased, highly | Increased, highly | Not measured |
| 2 | Skin, dermis | maintains | Decreased | Increased | Increased | Increased | Not measured |
| 3 | Tendon (Achilles, limb large, finger) | maintains | Decreased | Increased, highly | Increased, highly | Increased permeability | Not measured |
| 4 | Cartilage (ear, nose) | maintains | Decreased | Increased | Not measured | Increased | Not measured |
| 5 | Penis foreskin | maintains | Decreased, Highly | Increased, highly | Increased | Increased | Not measured |
| 6 | Penis, connective tissues | maintains | Decreased | Increased | Increased | Increased | Not measured |
| 7 | Liver | maintains | Decreased | Increased | Not measured | Increased | Decreased, cells more cohesive |
| 8 | Uterus | maintains | Decreased | Increased | Increased | Increased | Not measured |
| 9 | Ovaries | maintains | Decreased | Increased | Not measured | Increased | Not measured |
| 10 | Pancreas | maintains | Not measured | Not measured | Not measured | Increased permeability | Not identified |
| 11 | Pancreatic islets | maintains | Decreased | Not measured | Increased | Not measured | Decreased, cells remain cohesive |
| 12 | Eye lens | maintains | Not measured | Not identified | Not identified | Not identified | Decreased |
| 13 | Nerve | maintains | Decreased | Increased | Not measured | Not measured | Not measured |
| 14 | Muscle | maintains | Decreased, moderately | Not measured | Not measured | Not measured | Not measured |

### References:

### Articles

Carroll, E. P., J. S. Janicki, et al. (1989). "Myocardial stiffness and reparative fibrosis following coronary embolisation in the rat." Cardiovasc Res 23(8): 655-661.
Dechene, A., J. P. Sowa, et al. (2010). "Acute liver failure is associated with elevated liver stiffness and hepatic stellate cell activation." Hepatology 52(3): 1008-1016.
Discher, D. E., P. Janmey, et al. (2005). "Tissue cells feel and respond to the stiffness of their substrate." Science 310(5751): 1139-1143.
Engler, A. J., S. Sen, et al. (2006). "Matrix elasticity directs stem cell lineage specification." Cell 126(4): 677-689.
Engler, A. J., H. L. Sweeney, et al. (2007). "Extracellular matrix elasticity directs stem cell differentiation." J Musculoskelet Neuronal Interact 7(4): 335.
Friedman, S. L. (2010). "Evolving challenges in hepatic fibrosis." Nat Rev Gastroenterol Hepatol 7(8): 425-436.
Georges, P. C., J. J. Hui, et al. (2007). "Increased stiffness of the rat liver precedes matrix deposition: implications for fibrosis." Am J Physiol Gastrointest Liver Physiol 293(6): G1147-1154.
Hansen, G. A., J. D. Stitzel, et al. (2003). "Incidence of elderly eye injuries in automobile crashes: the effects of lens stiffness as a function of age." Annu Proc Assoc Adv Automot Med 47: 147-163.
Hirayasu, H., Y. Yoshikawa, et al. (2008). "A lymphocyte serine protease granzyme A causes detachment of a small-intestinal epithelial cell line (IEC-6)." Biosci Biotechnol Biochem 72(9): 2294-2302.
Hurst, L. C., M. A. Badalamente, et al. (2009). "Injectable collagenase clostridium histolyticum for Dupuytren's contracture." N Engl J Med 361(10): 968-979.
Klasen, H. J. (2000). "A review on the nonoperative removal of necrotic tissue from burn wounds." Burns 26(3): 207-222.
Koh, E. S., S. G. Chung, et al. (2012). "Changes in biomechanical properties of glenohumeral joint capsules with adhesive capsulitis by repeated capsule-preserving hydraulic distensions with saline solution and corticosteroid." PM R 4(12): 976-984.
Lehmann, R., R. A. Zuellig, et al. (2007). "Superiority of small islets in human islet transplantation." Diabetes 56(3): 594-603.
Li, D. S., Y. H. Yuan, et al. (2009). "A protocol for islet isolation from mouse pancreas." Nat Protoc 4(11): 1649-1652.
Lin, H. T., A. T. Hsu, et al. (2008). "Reliability of stiffness measured in glenohumeral joint and its application to assess the effect of end-range mobilization in subjects with adhesive capsulitis." Man Ther 13(4): 307-316.
Starkweather, K. D., S. Lattuga, et al. (1996). "Collagenase in the treatment of Dupuytren's disease: an in vitro study." J Hand Surg Am 21(3): 490-495.
Stitzel, J. D., G. A. Hansen, et al. (2005). "Blunt trauma of the aging eye: injury mechanisms and increasing lens stiffness." Arch Ophthalmol 123(6): 789-794.
Wang, J. H., C. S. Changchien, et al. (2009). "FibroScan and ultrasonography in the prediction of hepatic fibrosis in patients with chronic viral hepatitis." J Gastroenterol 44(5): 439-446.
Wang, M. H., M. L. Palmeri, et al. (2009). "In vivo quantification of liver stiffness in a rat model of hepatic fibrosis with acoustic radiation force." Ultrasound Med Biol 35(10): 1709-1721.
Wells, R. G. (2005). "The role of matrix stiffness in hepatic stellate cell activation and liver fibrosis." J Clin Gastroenterol 39(4 Suppl 2): S158-161.
"Matrix Metalloproteinase Protocols" by Clark, Ian M, published 2001, Methods in Molecular Biology, ISBN 1592590462, Vol. 151

### Patents

"Wound healing composition", US3003917 (A), 1961-10-10 by Beiler et.al.

US patent 6,365,405, by Salzmann et.al.(2002) "Compositions of chondrocytes, preparation and utilization"

US patent 8,323,642 by Story et al. (2012) "Tissue fusion method using collagenase for repair of soft tissue"

US patent application 20080145357 by Story et.al. (2008) "Tissue fusion method using collagenase for repair of soft tissue"

US patent application 20100247512, by Weiss et.al. (2010) "Pericellular Collagenase Directs the 3-Dimensional Development of White Adipose Tissue"

US patent 5,922,322, by Bini et.al. (1999) "Fibrin(ogen) degradation and clot lysis by fibrinolytic matrix metalloproteinase"

US patent application 20110091436, by Orbe et.al. (2011) "USE OF MATRIX METALLOPROTEINASE-10 (MMP-10) FOR THROMBOLYTIC TREATMENTS"

"Composition for revitalizing scar tissue", EP0637450 (A2); EP0637450 (A3), 1995-02-08, by Berg et.al.

"IN VIVO TEMPORAL CONTROL OF ACTIV AT ABLE MATRIX-DEGRADING ENZYMES", WO2009111083 (A2); WO2009111083 (A3), 2009-09-11, by Keller et.al.

US patent 7,935,337 by Haro (2011) "Method of therapy for degenerative intervertebral discs"

US patent application 20110044960 by Nakashima et.al. (2011) "MEDICAMENT, DENTAL MATERIAL, AND METHOD OF SCREENING"

## Claims

1. Use of pre-activated human MMP-9 to provide a modification of the mechanical properties of mammalian connective tissue while the tissue remains cohesive, wherein the MMP-9 has been pre-activated by 4-aminophenylmercuric acetate (APMA) activation and wherein the MMP-9 is incubated *ex vivo* with samples of mammalian connective tissue.

2. Use of pre-activated human MMP-9 according claim 1, wherein the connective tissue is cartilage.

3. Use of pre-activated human MMP-9 according to any one of the preceding claims, wherein the tissue retains cohesion when subjected to static or dynamic strain or deformation.

4. Use of pre-activated human MMP-9 according to any one of the preceding claims, wherein said mechanical properties is one or more property selected from the group consisting of stiffness, plasticity, elasticity, fragility, brittleness, mechanoelasticity, viscoelasticity, adhesiveness, ability to reduce risk of ruptures, ability to reduce risk of erosion or any stress-strain characteristics.

5. Use of pre-activated human MMP-9 according to any one of the preceding claims, wherein the modification of the mechanical properties is a decrease in stiffness (stress/strain ratio) of the tissue, preferably to over 1%, more preferably to over 10% and most preferably to over 30% in treated tissue as compared to non-treated.

6. Use of pre-activated human MMP-9 according to any one of the preceding claims, wherein the modification of the mechanical properties of the tissue is an increase of the limits of cohesive deformation (strain) or range of motion (ROM) of the tissue, preferably to over 1%, more preferably to over 10% and most preferably to over 30% in treated tissue as compared to non-treated.

7. Use of pre-activated human MMP-9 according to any one of the preceding claims, wherein the modification of the mechanical properties is an increase in the plasticity of the tissue, preferably to over 1%, more preferably to over 10% and most preferably to over 30% in treated tissue as compared to non-treated.

## Patentansprüche

1. Verwendung von voraktiviertem humanem MMP-9 zum Bereitstellen einer Modifizierung der mechanischen Eigenschaften von Säugetierbindegewebe, bei der das Gewebe kohäsiv bleibt, wobei das MMP-9 durch 4-Aminophenylquecksilberacetat-(APMA-)Aktivierung voraktiviert wurde und wobei das MMP-9 ex vivo mit Proben von Säugetierbindegewebe inkubiert wird.

2. Verwendung von voraktiviertem humanem MMP-9 nach Anspruch 1, wobei das Bindegewebe Knorpelgewebe ist.

3. Verwendung von voraktiviertem humanem MMP-9 nach einem der vorstehenden Ansprüche, wobei das Gewebe unter statischer oder dynamischer Belastung oder Deformierung Kohäsion bewahrt.

4. Verwendung von voraktiviertem humanem MMP-9 nach einem der vorstehenden Ansprüche, wobei die mechanischen Eigenschaften eine oder mehrere Eigenschaften, ausgewählt aus der Gruppe, bestehend aus Steifigkeit, Plastizität, Elastizität, Brüchigkeit, Sprödigkeit, mechanische Elastizität, Viskoelastizität, Adhäsionsvermögen, Fähigkeit, das Rissrisiko zu reduzieren, Fähigkeit, das Erosionsrisiko zu reduzieren, oder beliebigen Belastungs-/Beanspruchungseigenschaften, sind.

5. Verwendung von voraktiviertem humanem MMP-9 nach einem der vorstehenden Ansprüche, wobei die Modifizierung der mechanischen Eigenschaften eine Senkung der Steifigkeit (Belastungs-/Beanspruchungsverhältnis) des Gewebes, bevorzugt auf mehr als 1 %, mehr bevorzugt auf mehr als 10 % und am stärksten bevorzugt auf mehr als 30 %, in behandeltem Gewebe im Vergleich zu unbehandeltem Gewebe ist.

6. Verwendung von voraktiviertem humanem MMP-9 nach einem der vorstehenden Ansprüche, wobei die Modifizierung der mechanischen Eigenschaften des Gewebes eine Erhöhung der Grenzen der kohäsiven Deformierung (Belastung) oder des Bewegungsbereichs (range of motion - ROM) des Gewebes, bevorzugt auf mehr als 1 %, mehr bevorzugt auf mehr als 10 % und am stärksten bevorzugt auf mehr als 30 %, in behandeltem Gewebe im Vergleich zu unbehandeltem Gewebe ist.

7. Verwendung von voraktiviertem humanem MMP-9 nach einem der vorstehenden Ansprüche, wobei die Modifizierung der mechanischen Eigenschaften eine Erhöhung der Plastizität des Gewebes, bevorzugt auf mehr als 1 %, mehr bevorzugt auf mehr als 10 % und am stärksten bevorzugt auf mehr als 30 %, in behandeltem Gewebe im Vergleich zu unbehandeltem Gewebe ist.

## Revendications

1. Utilisation de MMP-9 humaine pré-activée donnant lieu à une modification des propriétés mécaniques du tissu conjonctif de mammifère, alors même que les tissus restent homogènes, dans laquelle la MMP-9 a été pré-activée grâce à l'activation de l'acétate 4-aminophénylmercurique (APMA) et dans laquelle la MMP-9 est incubée *ex vivo* avec des échantillons de tissu conjonctif de mammifère.

2. Utilisation de MMP-9 humaine pré-activée selon la revendication 1, dans laquelle le tissu conjonctif est du cartilage.

3. Utilisation de MMP-9 humaine pré-activée selon l'une quelconque des revendications précédentes, dans laquelle le tissu reste homogène lorsqu'il est soumis à des contraintes ou déformations statiques ou dynamiques.

4. Utilisation de MMP-9 humaine pré-activée selon l'une quelconque des revendications précédentes, dans laquelle lesdites propriétés mécaniques se traduisent par une ou plusieurs propriétés appartenant à un groupe constitué de rigidité, plasticité, élasticité, fragilité, friabilité, mécano-sensibilité, viscoélasticité, adhérence, capacité à limiter le risque de rupture, capacité à réduire le risque d'érosion ou toute autre caractéristique de tension-dilatation.

5. Utilisation de MMP-9 humaine pré-activée selon l'une quelconque des revendications précédentes, dans laquelle les modifications des propriétés mécaniques se traduisent par une diminution de la rigidité des tissus (rapport tension-dilatation), préférablement au-delà de 1 %, plus préférablement au-delà de 10 %, et le plus préférablement au-delà de 30 % dans les tissus traités par rapport aux tissus non-traités.

6. Utilisation de MMP-9 humaine pré-activée selon l'une quelconque des revendications précédentes, dans laquelle les modifications des propriétés mécaniques du tissu se traduisent par une augmentation des limites de la déformation homogène (tension) ou une amplitude des mouvements (ADM) des tissus, préférablement au-delà de 1 %, plus préférablement au-delà de 10 %, et le plus préférablement au-delà de 30 % dans les tissus traités par rapport aux tissus non-traités.

7. Utilisation de MMP-9 humaine pré-activée selon l'une quelconque des revendications précédentes, dans laquelle les modifications des propriétés mécaniques se traduisent par une augmentation de la plasticité des tissus, préférablement au-delà de 1 %, plus préférablement au-delà de 10 %, et le plus préférablement au-delà de 30 % dans les tissus traités par rapport aux tissus non-traités.
